# EUROPEAN PATENT SPECIFICATION

(11) **EP 2 836 203 B1**
(45) Date of publication and mention of the grant of the patent: **09.03.2022**
(21) Application number: 13715989.3
(22) Date of filing: 12.04.2013
(51) Int. Cl.: A61K 31/661, A61K 31/683, A61P 17/00, A61P 19/02, A61P 29/00, A61K 9/127, A61K 9/107, A61K 31/688, A61K 31/685, A61K 47/24, A61K 9/00

(54) **VESICULAR FORMULATIONS FOR USE IN THE TREATMENT OF PAIN OR REDUCED MOBILITY OF A JOINT**
VESIKULÄRE FORMULIERUNGEN ZUR ANWENDUNG IN DER BEHANDLUNG VON SCHMERZ ODER VERMINDERTER BEWEGLICHKEIT EINES GELENKES
FORMULATIONS VÉSICULAIRES POUR UTILISATION DANS LE TRAITEMENT DE LA DOULEUR OU DE LA MOBILITÉ RÉDUITE D'UNE ARTICULATION

(30) Priority: 12.04.2012 GB 201206486
(43) Date of publication of application: 18.02.2015
(73) Proprietor: Sequessome Technology Holdings Limited, Valletta 1436 (MT)
(72) Inventor: GARRAWAY, Richard, Wolf, London Greater London WC1B 3RA (GB); EARL, Michael, London Greater London WC1B 3RA (GB); YURDAKUL, Saruhan, London Greater London WC1B 3RA (GB); BAVERSTOCK, Nicholas, London Greater London WC1B 3RA (GB)
(74) Representative: Lee, Nicholas John
(86) International application number: PCT/EP2013/057742
(87) International publication number: WO 2013/153221

(56) References cited:
- WO-A1-2011/022707
- WO-A2-2010/140061
- US-A- 5 853 753
- US-A1- 2005 123 593
- US-A1- 2010 098 749
- KAWANO T ET AL: "Mechanical effects of the intraarticular administration of high molecular weight hyaluronic acid plus phospholipid on synovial joint lubrication and prevention of articular cartilage degeneration in experimental osteoarthritis", ARTHRITIS & RHEUMATISM, JOHN WILEY & SONS, INC, US, vol. 48, no. 7, 1 July 2003 (2003-07-01), pages 1923-1929, XP002301692, ISSN: 0004-3591, DOI: 10.1002/ART.11172

## Description

The present invention relates to vesicular formulations for use in the treatment of reduced mobility associated with a loss of lubrication and/or structural integrity and/or swelling of a collagen structure. Also described herein is a method of treating pain such as joint pain or tendonitis comprising topically administering a vesicular formulation for use according to the invention.

In healthy joints (such as the knee), the bones are not directly connected but are joined by connective tissue that forms a capsule around the ends of the bones. A layer of cartilage (which comprises collagen as the major tissue, as well as proteoglycans and elastins) covers the ends of the bones within the joint. The area between the bones, within the capsule, is filled with synovial fluid. The surface of the cartilage layer found on the end of each bone is coated with a layer of phospholipids which are anchored within the cartilage structure. The hydrophilic head of each molecule is orientated into the synovial space and each is highly hydrated, which allows free movement of the joint without pain or discomfort. As the phospholipids on the cartilage surface deplete, in a healthy joint they are replenished by phospholipids present in the synovial fluid. Articular cartilage as well as other collagenous material such as ligaments and tendons, consist of a matrix of structural molecules and cellular material. One integral structural molecule is proteoglycan which is highly hydrated due to the molecule being highly sulphated. The hydroscopic nature of these structural subunits gives cartilage and other collagenous materials high tensile strength.

In a damaged joint, for example due to osteoarthritis, overuse, excess weight etc, inflammation occurs. One factor of the inflammation cascade is an increase in phospholipases. These enzymes break down the phospholipids on the cartilage surface and in the synovial fluid, leading to the cleavage of the lipids and thus degradation of the protective layer between the layers of cartilage. This loss of protection leads to further progressive damage of the cartilage by way of erosion and friction, which leads to pain and/or reduced mobility of the joint. In osteoarthritis (OA), for example, the proteoglycan molecules (a key factor of the structural integrity) break down and become fragmented. This degradation reduces the water capacity and therefore water content of the collagenous components of the joint thereby losing structural support. Through this mechanism the structural integrity of various joint structures including articular cartilage, ligaments, joint membranes etc is compromised causing various OA symptoms, including pain, loss of mobility and swelling. Joint degradation causes pain via the release of pain mediators from destroyed cells. Loss of structural integrity contributes to further cellular destruction and, macroscopically, joint degradation, both factors increasing the release of pain mediators.

Currently, either the pain is managed by way of analgesics and/or anti-inflammatory medication. Alternatively, hyaluronic acid, a polymer can be injected on a regular basis in to the synovial capsule to provide enhanced temporary lubrication of the joint. However, the procedure is painful in itself, involving the injection of the polymer using a large bore needle directly into the joint. The procedure must be repeated on a monthly or up to three monthly basis and can only be carried out by a specialised physician or other medical professional. Clearly this is far from ideal in managing joint pain on a long term basis.

Joint pain and/or reduced mobility may also be due to a loss of lubrication and/or structural integrity and/or swelling due to the degradation or lack of a protein called lubricin, a proteoglycan thought to assist in the free movement of joints, which is found in the synovial fluid. The vesicular formulation used in accordance with the invention can provide lubrication and thus pain relief, when lubricin is depleted. The formulation for use according to the invention can provide an increase in joint mobility.

Carpal tunnel syndrome can be caused by inflammation in the wrist, causing pressure to build up around the nerves. The present invention can provide lubrication around the tendons and ligaments of the wrist, allowing the inflammation to subside and reduce the pressure on the nerves, and thus reducing pain, and increasing mobility.

It is also known that specialised liposomes can provide a lubricating film to mitigate the effects of inflammatory damage. (Sivan, S. et al (2010) Longmuir, 26(2), 1107-1116). However, these would require intra-articular injection to deliver the liposomes to the site of damage. This would be a monthly procedure in all likelihood. The procedure is the same as described above, and therefore both inconvenient and painful for the patient. The liposomes, in order to be effective, must also be highly hydrated, highly compressible and very stable. Vesicles lacking any one of these properties will not provide suitable lubrication to overcome the lack of natural lubrication in the inflamed joint.

Therefore, present invention provides a vesicular formulation consisting essentially of one or more phospholipids, one or more non-ionic surfactants and a pharmaceutically acceptable carrier, and optionally further containing one or more of the following ingredients: co-solvents, chelators, buffers, antioxidants, preservatives, microbicides, emollients, humectants, lubricants and thickeners, for use in the treatment of reduced mobility associated with a loss of lubrication and/or structural integrity and/or swelling of a collagen structure in an animal by topically applying the vesicular formulation to the skin surrounding the collagen structure.

Also described herein is a vesicular formulation comprising a phospholipid and a surfactant for use in the treatment of pain and/or reduced mobility associated with a loss of lubrication as well as a loss of structural integrity and/or swelling of a collagen structure in an animal by topically applying the vesicular formulation to the skin surrounding the collagen structure. The collagen structure may be cartilage within an articulated joint or it may be a tendon or a ligament.

The vesicular formulation for use according to the invention may be useful in the prevention or treatment of joint locking or joint freezing (i.e. immobility of a joint) associated with a loss of lubrication and/or structural integrity and/or swelling of a collagen structure in an animal.

It is appreciated by the skilled person that pain due to a loss of lubrication and/or structural integrity and/or swelling may be treated independently of reduced mobility due to a loss of lubrication and/or structural integrity and/or swelling. The present invention is concerned with the treatment of pain, of reduced mobility or a combination of pain and reduced mobility. Improving the level of pain may result in increased mobility and vice versa. A loss of lubrication and/or structural integrity and/or swelling may result in a loss of mobility but little pain or vice versa. Thus, the present invention may be useful for the treatment of pain and/or reduced mobility.

The vesicular formulation for use according to the invention may treat pain and/or reduced mobility due to a loss of lubrication or due to a loss of structural integrity or a combination of loss of lubrication and structural integrity. In other words, the pain and/or reduced mobility may be treated by addressing the loss of lubrication or by addressing the loss of structural integrity with the use of the vesicular formulation.

The loss of lubrication and/or structural integrity and/or swelling may be due to degradation of phospholipids upon the surface of cartilage within an articulated joint or due to the depletion of phospholipids within the synovial fluid in an articulated joint.

In the case of a tendon or a ligament, the loss of lubrication and/or structural integrity and/or swelling may be due to depletion of phospholipids between the tendon or ligament and a surface over which it moves.

In the present invention, reduced mobility is treated by visco-supplementation.

Described herein is a vesicular formulation comprising a phospholipid and a surfactant for use in the treatment of joint pain and/or reduced mobility. The joint pain and/or reduced mobility may be associated with a reduction in phospholipids in the synovial fluid and/or with osteoarthritis or rheumatoid arthritis. The joint may be a knee, hip, shoulder, elbow, wrist, ankle, hand, finger, toe, foot or other point of articulation, for example between vertebrae and the intervertebral discs.

The invention also provides a vesicular formulation comprising a phospholipid and a surfactant for use in the treatment of tendonitis or for the treatment of carpal tunnel syndrome.

The animal to be treated may be a human, a companion animal or an agricultural animal. Anecdotal evidence suggests that arthritis in cats and dogs may be successfully treated in accordance with the invention.

When the animal to be treated is a human, the invention may be useful for those patients aged 45 to 85 years old, 50 to 80 years old, 55 to 75 years old or 60 to 65 years old. The human patient may be male or female.

Also described herein is a method of treating pain and/or reduced mobility associated with a loss of lubrication and/or structural integrity and/or swelling of a collagen structure comprising topically applying a vesicular formulation comprising a phospholipid and a surfactant to the skin surrounding the collagen structure is provided.

Also described herein is a method of treating osteoarthritis, carpal tunnel syndrome or tendonitis comprising topically applying a vesicular formulation comprising a phospholipid and a surfactant to the skin surrounding the collagen structure.

All features of the first aspect apply to the second and third aspects of the invention, *mutatis mutandis.*

US Patent No. 6,165,500 describes a preparation for the application of agents which are provided with membrane-like structures consisting of one or several layers of amphiphilic molecules, or an amphiphilic carrier substance, in particular for transporting the agent into and through natural barriers such as skin and similar materials. These Transfersome^{™} formulations consist of one or several components, most commonly a mixture of basic substances, one or several edge-active substances, and agents.

US Patent Application Publication No. US 2004/0071767 describes formulations of nonsteroidal anti-inflammatory drugs (NSAIDs) based on complex aggregates with at least three amphiphatic components suspended in a pharmaceutically acceptable medium.

US Patent Application Publication No. US 2004/0105881 describes extended surface aggregates, suspendable in a suitable liquid medium and comprising at least three amphiphats (amphiphatic components) and being capable to improve the transport of actives through semi-permeable barriers, such as the skin, especially for the non-invasive drug application *in vivo* by means of barrier penetration by such aggregates. WO 2010/140061 describes the use of "empty" vesicular formulations for the treatment of deep tissue pain. WO 2011/022707 describes the use of the same "empty" vesicles for treating disorders relating to fatty acid deficiencies and *inter alia* disorders related to inflammation.

None of these documents disclose or teach the use of vesicular formulations for the treatment of pain and/or reduced mobility caused by a loss of lubrication and/or structural integrity and/or swelling of a collagen structure.

Citation of any reference in this section of the application is not an admission that the reference is prior art to the application.

The present invention relates to a vesicular formulation comprising a phospholipid and a surfactant for use in the treatment of reduced mobility associated with a loss of lubrication and/or structural integrity and/or swelling of a collagen structure. Vesicular formulations are described in WO2011/022707 and WO2010/140061 and throughout this application. The formulation may be a cream, lotion, ointment, gel, solution, spray, lacquer or film forming solution.

The vesicular formulation does not need to contain any known pharmaceutically active ingredient. The formulation may not contain any known pharmaceutically active ingredient acknowledged in the prevention or treatment of pain, reduced mobility or inflammation.

The present invention is useful in a method of treating reduced mobility due to a loss of lubrication and/or structural integrity and/or swelling of a collagen structure, the method comprising topically administering a formulation according to the invention to the skin surrounding the collagen structure in a patient in need thereof.

Described herein are vesicular formulations comprising one or more phospholipids and one or more surfactants that are effective for the delivery of fatty acids and/or phospholipids in the treatment of pain and/or reduced mobility caused by a loss of lubrication and/or structural integrity and/or swelling of a collagen structure. These vesicular formulations are suitable for topical administration.

The formulations described herein are preferably formulated in the absence of any pharmaceutically active agent, i.e., any non-lipid non- surfactant pharmaceutically active agent. However, it may be possible to include an active agent within the structural wall or lumen of the vesicles of the formulation. Such agents may include an analgesic, an anti-inflammatory, a steroid or therapeutic proteins.

A pharmaceutically active agent is here defined as an agent that has pharmacological, metabolic or immunological activity.

Despite the lack of a recognized pharmaceutically active agent, the vesicles elicit a therapeutic effect, namely the treatment of pain and/or reduced mobility associated with a loss of lubrication and/or structural integrity and/or swelling of a collagen structure. Without being bound by any theory, the applicant believes that the vesicle components themselves are responsible for this effect.

The vesicular formulation for use in the invention consists essentially of one or more phospholipids and one or more surfactants and a pharmaceutically acceptable carrier.

The vesicular formulation for use according to the invention may optionally contain one or more of the following ingredients: co-solvents, chelators, buffers, antioxidants, preservatives, microbicides, emollients, humectants, lubricants and thickeners.

The vesicular formulation for use according to the invention is able (without wishing to be bound by theory) to achieve its function through the unique properties of multilayer vesicles, bilayer vesicles, micelles or aggregates composed of surfactant and lipid ("vesicles"), such as soy phosphatidylcholine. The uniqueness of the vesicles derives from the inclusion in the formulation of a specific amount of surfactant, which modifies the phospholipid membrane to such an extent that the resulting vesicles are in a permanent liquid crystalline state and, since the surfactant also confers membrane stability, the vesicles are ultra hydrated, deformable and stable (have reduced rigidity without breaking). The surfactant may be nonionic.

The vesicular formulation contains vesicles suspended in an aqueous buffer that is applied topically. The vesicles are highly hydrophilic and this property, together with their ultra deformability, is key to their ability to be transported across the skin and into the tissue to lubricate collagen structures. When the formulation for use according to the invention is applied to the skin and allowed to dry, the rehydration driving force of the vesicles combined with their deformability gives rise to movement of the vesicles to areas of higher water content on and below the skin permeability barrier. This drives their movement through skin pores and intracellular gaps. The specific ratio of lipid to surfactant facilitates transdermal delivery of vesicles.

Once they pass through the skin, the vesicles for use according to the invention (sometimes referred to as "Deformasomes") eventually present as intact vesicles. Efficient clearance of vesicles does not occur via the cutaneous blood microvasculature (capillaries) owing to their relatively large size, but they are hypothesised to be transported with the interstitial fluid into other and/or deeper tissues below the site of dermal application. A number of clinical studies conducted with vesicles for use according to the invention labelled with a marker molecule (terbinafine) showed that the vesicles did not enter the vasculature because, following topical application, high concentrations of the marker molecule were observed locally with minimal systemic absorption (at or below the level of detection). Due to highly deformable nature of the vesicles, they are able to penetrate the tissue from the skin through to the synovial capsule or other collagen containing structures. Here, the vesicles deform into a flattened lamellar-like structure to present the highly hydrophilic and hydrated head groups of the phospholipids to the surface of the collagen structure, be that within a joint in the form of cartilage or a tendon or ligament, on the surface of or within bones, (as in carpal tunnel syndrome).

Again, without wishing to be bound by theory, the loss of lubrication and/or structural integrity may be addressed by the replacement of lost membrane components. The vesicles for use according to the invention may be able to repair synovial or protective membranes around a collagen structure (such as cartilage or tendons/ligaments, respectively) by providing structural support to prevent further loss of lubricating, structural or protective joint constituents or tendon/ligament associated factors.

The vesicles of the vesicular formulation for use in the invention are highly hydrated and are able to penetrate into the porous collagenous structures of the joints. Once occupying the extracellular matrix the vesicles can provide hydration and support to the collagenous material thus restoring the joint integrity lost as a consequence of, for example, OA.

Restoration of structural integrity of cartilage and other collagenous joint structures may reduce symptoms such as pain while restoring the integrity of boundary structures such as the synovial membrane may reduce effusion and subsequently joint swelling.

Furthermore, the vesicles for use according to the invention may penetrate a collagen matrix (e.g. the cartilaginous cap of a long bone) to provide a nano-scaffold or nano-support to such a collagen structure that has lost its structural integrity.

Also described herein is a pharmaceutical package or kit comprising one or more containers filled with the formulation for use according to the invention, and instructions for administration of the formulation to a patient or subject in need thereof for the treatment of pain and/or reduced mobility associated with a lack of lubrication. In various embodiments, the container comprises a formulation formulated as a suspension, emulsion, gel, cream, lotion, spray, film forming solution or lacquer. The packages or kits that can be used in any of the above- described methods or uses.

Also described herein is a method for the treatment of pain and/or reduced mobility associated with a lack of lubrication of collagen structures, wherein the vesicular formulations are topically administered over a period of one or more weeks, for example for at least five weeks, six weeks, seven weeks, eight weeks, nine weeks, ten weeks, eleven weeks, or twelve weeks, sixteen weeks, twenty four weeks, four months, six months, eight months, ten months, one year, two or more years, or indefinitely. The formulation may be administered once, twice, three times or more per day. Alternatively the formulation may be administered on alternate days, two or three times per week, once per week or less frequently as needed.

In one embodiment, a 0.1 to 10 gram dose of the formulation for use according to the invention is administered to the patient. The dose may be 1 to 10 gram, or 1 to 5 gram or about 1 gram, 2 gram, 3 gram, 4 gram, 5 gram, 6 gram, 7 gram, 8 gram, 9 gram or 10 gram. In some embodiments, the dose is measured as the total weight of the Deformasome. In some embodiments, the dose is measured as the total weight of the lipid(s) and surfactant(s) in the Deformasome. The dose may be administered once or twice daily for the treatment of pain associated with loss of lubrication and/or structural integrity and/or swelling. The dose may be administered once, twice, three, four, five, six, or seven times per week in accordance with the invention. The dose may be administered every day, every other day, or two to three times a week in accordance with the invention.

The formulation is topically applied to the skin surrounding collagen structure where pain is felt by the patient. For example where the pain is joint pain and the reduced mobility is joint stiffness, the skin surrounding the knee joint, wrist, shoulder, ankle, hip, elbow or back. Alternatively the formulation can be applied to the skin of the shin, thigh, lower or upper arm, back etc in the case of pain due to tendonitis. The formulation may be applied to any external skin surface.

In some embodiments, the lipid in the pharmaceutical composition is a phospholipid. In some embodiments, the second lipid is a lysophospholipid. In some embodiments, the surfactant is a nonionic surfactant.

In some embodiments, the compositions for use according to the invention form vesicles or other extended surface aggregates (ESAs), wherein the vesicular preparations have improved permeation capability through the semi-permeable barriers, such as skin. The adaptability and deformability of the vesicles allow the vesicles to penetrate beneath the skin to the muscle and the joint itself, however, the size of the vesicle prevents penetration into the vasculature and as a result prevents systemic delivery. While not to be limited to any mechanism of action, the formulations for use according to the invention are able to form vesicles characterized by their deformability and/or adaptability. The adaptability or deformability of the vesicles may be determined by the ability of the vesicles to penetrate a barrier with pores having an average pore diameter that is smaller than the average vesicle diameter before the penetration. The pore diameter may be at least 50% smaller than the average vesicle diameter.

Generally, the nomenclature used herein and the laboratory procedures in organic chemistry, medicinal chemistry, and pharmacology described herein are those well known and commonly employed in the art. Unless defined otherwise, all technical and scientific terms used herein generally have the same meaning as commonly understood by one of ordinary skill in the art to which this disclosure belongs.

The invention can be used on any animal which suffers from pain or reduced mobility caused by a lack of lubrication of a collagen structure. For example, the invention is useful for the treatment of humans, or a companion animal (e.g. a cat, a dog or a horse) or an agricultural animal.

As used herein, a "sufficient amount." "amount effective to" or an "amount sufficient to" achieve a particular result refers to an amount of the formulation of the invention is effective to produce a desired effect, which is optionally a therapeutic effect (i.e., by administration of a therapeutically effective amount). Alternatively stated, a "therapeutically effective" amount is an amount that provides some alleviation, mitigation, and/or decrease in at least one clinical symptom. Clinical symptoms associated with the disorder that can be treated by the methods of the invention are well-known to those skilled in the art. Further, those skilled in the art will appreciate that the therapeutic effects need not be complete or curative, as long as some benefit is provided to the subject. For example, a "sufficient amount" or "an amount sufficient to" can be an amount that is effective to treat the symptoms of pain associated with a lack of lubrication of a collagen structure.

As used herein, the term "reduced mobility" refers to reduced joint mobility as compared to a healthy joint, when the collagen structure is within an articulated joint. This can also be referred to as "joint stiffness". The invention allows for improvement in mobility and stiffness such that it is easier to move the joint affected by a loss of lubrication and/or structural integrity and/or swelling, upon administration of the vesicular formulation. Thus, physical function of a joint is improved. The vesicular formulation for use according to the invention can prevent joint locking or joint freezing, which may occur if the reduced mobility is left untreated. Joint locking or freezing, as used herein, means the inability to move or bend the joint in question. Where the collagen structure is a tendon or ligament, reduced mobility may also be due to pain or discomfort associated with tendonitis, or a ligament wherein the pain is not localised to or associated with a specific joint.

As used herein, pain or reduced mobility due to a loss of lubrication means that the pain or reduced mobility is associated with friction within a joint due to a degradation of cartilage of the joint, or the friction caused by degradation of collagen or protective elements/membranes of a tendon or ligament.

As used herein, the term "structural integrity" means the integrity of articular cartilage, or synovial membranes, or membranes surrounding tendons or ligaments. A loss of structural integrity is used herein to mean that the collagen structure to which this term relates has been compromised, for example, by the loss or degradation of one or more structural proteins (such as proteoglycan) or the loss or degradation of one or more membrane components (such as phospholipids). When referring to joints, loss of integrity may be associated with the destruction of cartilage, beginning with breakdown and release of proteoglycan, leading to destruction of collagen type II and complete or partial loss of the cartilage matrix. Due to the slow growing nature of cartilage, degradation usually happens at a greater rate, leading to the net reduction of cartilage structures in affected joints. The vesicular formulation for use according to the present invention therefore allows the body's natural healing process to "catch up" by providing support to cartilage matrix, preventing further degradation.

Swelling as used in the context of the invention refers to swelling via effusion (fluid leak into the joint), which can occur when the structural integrity of the synovial membrane is compromised. Increasing the fluid content of the joint increases the hydrostatic pressure further progressing damage to the joint.

As used herein, the terms "treat", "treating" or "treatment of mean that the severity of a subject's condition is reduced or at least partially improved or ameliorated and/or that some alleviation, mitigation or decrease in at least one clinical symptom is achieved and/or there is an inhibition or delay in the progression of the condition and/or delay in the progression of the onset of disease or illness. The terms "treat", "treating" or "treatment of also means managing the disease state.

As used herein, the term "pharmaceutically acceptable" when used in reference to the formulations for use according to the invention denotes that a formulation does not result in an unacceptable level of irritation in the subject to whom the formulation is administered. Preferably such level will be sufficiently low to provide a formulation suitable for approval by regulatory authorities.

As used herein with respect to numerical values, the term "about" means a range surrounding a particular numeral value which includes that which would be expected to result from normal experimental error in making a measurement. For example, in certain embodiments, the term "about" when used in connection with a particular numerical value means +-20%, unless specifically stated to be +-1%, +-2%, +-3%, +-4%, +-5%, +-10%. +-15%, or +-20% of the numerical value.

The term "alkyl" refers to a linear or branched saturated monovalent hydrocarbon radical, wherein the alkyl may optionally be substituted with one or more substituents Q as described herein. The term "alkyl" also encompasses both linear and branched alkyl, unless otherwise specified. In certain embodiments, the alkyl is a linear saturated monovalent hydrocarbon radical that has 1 to 20 (C₁₋₂₀), 1 to 15 (C₁₋₁₅), 1 to 12 (C₁₋₁₂), 1 to 10 (C₁₋₁₀), or 1 to 6 (C₁₋₆) carbon atoms, or a branched saturated monovalent hydrocarbon radical of 3 to 20 (C₃₋₂₀), 3 to 15 (C₃₋₁₅), 3 to 12 (C₃₋₁₂), 3 to 10 (C₃₋₁₀), or 3 to 6 (C₃₋₆) carbon atoms. As used herein, linear C₁₋₆ and branched C₃₋₆ alkyl groups are also referred as "lower alkyl". Examples of alkyl groups include, but are not limited to, methyl, ethyl, propyl (including all isomeric forms), n-propyl, isopropyl, butyl (including all isomeric forms), n-butyl, isobutyl, sec-butyl, t-butyl, pentyl (including all isomeric forms), and hexyl (including all isomeric forms). For example, C₁₋₆ alkyl refers to a linear saturated monovalent hydrocarbon radical of 1 to 6 carbon atoms or a branched saturated monovalent hydrocarbon radical of 3 to 6 carbon atoms. It is understood in the chemical arts, that the use of the longer chains described herein may be appropriate, or appropriate only in limited amounts, within a molecule so that the properties of the resulting molecule (such as solubility) are appropriate for the use. Thus, while those in the art may use the above longer length alkyl substituents they will be used only when appropriate to provide the desired function.

The term "aryl" refers to a monocyclic aromatic group and/or multicyclic monovalent aromatic group that contain at least one aromatic hydrocarbon ring. In certain embodiments, the aryl has from 6 to 20 (C₆₋₂₀), from 6 to 15 (C₆₋₁₅), or from 6 to 10 (C₆₋₁₀) ring atoms. Examples of aryl groups include, but are not limited to, phenyl, naphthyl, fluorenyl, azulenyl, anthryl, phenanthryl, pyrenyl, biphenyl, and terphenyl. Aryl also refers to bicyclic or tricyclic carbon rings, where one of the rings is aromatic and the others of which may be saturated, partially unsaturated, or aromatic, for example, dihydronaphthyl, indenyl, indanyl, or tetrahydronaphthyl (tetralinyl). In certain embodiments, aryl may also be optionally substituted with one or more substituents Q as described herein.

The term "heteroaryl" refers to a monocyclic aromatic group and/or multicyclic aromatic group that contain at least one aromatic ring, wherein at least one aromatic ring contains one or more heteroatoms independently selected from O, S. and N. Each ring of a heteroaryl group can contain one or two O atoms, one or two S atoms, and/or one to four N atoms, provided that the total number of heteroatoms in each ring is four or less and each ring contains at least one carbon atom. The heteroaryl may be attached to the main structure at any heteroatom or carbon atom which results in the creation of a stable compound. In certain embodiments, the heteroaryl has from 5 to 20, from 5 to 15, or from 5 to 10 ring atoms. Examples of monocyclic heteroaryl groups include, but are not limited to, pyrrolyl, pyrazolyl, pyrazolinyl, imidazolyl, oxazolyl, isoxazolyl, thiazolyl, thiadiazolyl, isothiazolyi. furanyl. thienyl, oxadiazolyl, pyridyl, pyrazinyl, pyrimidinyl, pyridazinyl, and triazinyl. Examples of bicyclic heteroaryl groups include, but are not limited to, indolyl, benzothiazolyl, benzoxazolyl, benzothienyl, quinolinyl, tetrahydroisoquinolinyl, isoquinolinyl, benzimidazolyl, benzopyranyl, indolizinyl, benzofuranyl, isobenzofuranyl, chromonyl, coumarinyl, cinnolinyl, quinoxalinyl, indazolyl, purinyl, pyrrolopyridinyl, furopyridinyl, thicnopyridinyl, dihydroisoindolyl, and tetrahydroquinolinyl. Examples of tricyclic heteroaryl groups include, but are not limited to carbazolyl, benzindolyl, phenanthrollinyl, acridinyl, phenanthridinyl, and xanthenyl. In certain embodiments, heteroaryl may also be optionally substituted with one or more substituents Z as described herein.

The term "alkenoyl" as used herein refers to -C(O)-alkenyl. The term "alkenyl" refers to a linear or branched monovalent hydrocarbon radical, which contains one or more, in one embodiment, one to five, carbon-carbon double bonds. The alkenyl may be optionally substituted with one or more substituents Z as described herein. The term "alkenyl" also embraces radicals having "*cis*" and "*trans*" configurations, or alternatively, "Z" and "E" configurations, as appreciated by those of ordinary skill in the art. As used herein, the term "alkenyl" encompasses both linear and branched alkenyl, unless otherwise specified. For example, C₂₋₆ alkenyl refers to a linear unsaturated monovalent hydrocarbon radical of 2 to 6 carbon atoms or a branched unsaturated monovalent hydrocarbon radical of 3 to 6 carbon atoms. In certain embodiments, the alkenyl is a linear monovalent hydrocarbon radical of 2 to 30 (C₂₋₃₀), 2 to 24 (C₂₋₂₄), 2 to 20 (C₂₋₂₀), 2 to 15 (C₂₋₁₅), 2 to 12 (C₂₋₁₂), 2 to 10 (C₂₋₁₀), or 2 to 6 (C₂₋₆) carbon atoms, or a branched monovalent hydrocarbon radical of 3 to 30 (₃₋₃₀), 3 to 24 (C₃₋₂₄). 3 to 20 (C₃₋₂₀), 3 to 15 (C₃₋₁₅), 3 to 12 (C₃₋₁₂), 3 to 10 (C₃₋₁₀), or 3 to 6 (C₃₋₆) carbon atoms. Examples of alkenyl groups include, but are not limited to, ethenyl, propen-1-yl, propen-2-yl, allyl, butenyl, and 4-methylbutenyl. In certain embodiments, the alkenoyl is mono-alkenoyl, which contains one carbon-carbon double bond. In certain embodiments, the alkenoyl is di-alkenoyl, which contains two carbon-carbon double bonds. In certain embodiments, the alkenoyl is poly-alkenoyl, which contains more than two carbon-carbon double bonds.

The term "heterocyclyl" or "heterocyclic" refers to a monocyclic non-aromatic ring system and/or multicyclic ring system that contains at least one non-aromatic ring, wherein one or more of the non-aromatic ring atoms are heteroatoms independently selected from O, S, or N; and the remaining ring atoms are carbon atoms. In certain embodiments, the heterocyclyl or heterocyclic group has from 3 to 20, from 3 to 15, from 3 to 10, from 3 to 8, from 4 to 7, or from 5 to 6 ring atoms. In certain embodiments, the heterocyclyl is a monocyclic, bicyclic, tricyclic, or tetracyclic ring system, which may include a fused or bridged ring system, and in which the nitrogen or sulfur atoms may be optionally oxidized, the nitrogen atoms may be optionally quaternized, and some rings may be partially or fully saturated, or aromatic. The heterocyclyl may be attached to the main structure at any heteroatom or carbon atom which results in the creation of a stable compound. Examples of such heterocyclic radicals include, but are not limited to, acridinyl, azepinyl, benzimidazolyl, benzindolyl, benzoisoxazolyl, benzisoxazinyl, benzodioxanyl, benzodioxolyl, benzofuranonyl, benzofuranyl, benzonaphthofuranyl, benzopyranonyl, benzopyranyl, benzotetrahydrofuranyl, benzotetrahydrothienyl, benzothiadiazolyl, benzothiazolyl, benzothiophenyl, benzotriazolyl, benzothiopyranyl, benzoxazinyl, benzoxazolyl, benzothiazolyl, [beta]-carbolinyl, carbazolyl, chromanyl, chromonyl, cinnolinyl, coumarinyl, decahydroisoquinolinyl, dibenzofuranyl, dihydrobenzisothiazinyl. dihydrobenzisoxazinyl, dihydrofuryl, dihydropyranyl, dioxolanyl, dihydropyrazinyl, dihydropyridinyl, dihydropyrazolyl, dihydropyrimidinyl, dihydropyrrolyl, dioxolanyl, 1,4-dithianyl, furanonyl, furanyl, imidazolidinyl, imidazolinyl, imidazolyl, imidazopyridinyl, imidazothiazolyl, indazolyl, indolinyl, indolizinyl, indolyl, isobenzotetrahydro furanyl, isobenzotetrahydrothienyl, isobenzothienyl, isochromanyl, isocoumarinyl, isoindolinyl, isoindolyl, isoquinolinyl, isothiazolidinyl, isothiazolyl, isoxazolidinyl, isoxazolyl, morpholinyl, naphthyridinyl, octahydroindolyl, octahydroisoindolyl, oxadiazolyl, oxazolidinonyl, oxazolidinyl, oxazolopyridinyl, oxazolyl, oxiranyl, perimidinyl, phenanthridinyl, phenathrolinyl, phenarsazinyl, phenazinyl, phenothiazinyl, phenoxazinyl, phthalazinyl, piperazinyl, piperidinyl, 4-piperidonyl, pteridinyl, purinyl, pyrazinyl, pyrazolidinyl, pyrazolyl, pyridazinyl, pyridinyl, pyridopyridinyl, pyrimidinyl, pyrrolidinyl, pyrrolinyl, pyrrolyl, quinazolinyl. quinolinyl, quinoxalinyl, quinuclidinyl, tetrahydrofuryl, tetrahydro furanyl, tetrahydroisoquinolinyl, tetrahydropyranyl, tetrahydrothienyl, tetrazolyl, thiadiazolopyrimidinyl, thiadiazolyl, thiamorpholinyl, thiazolidinyl, thiazolyl, thienyL triazinyl, triazolyl, and 1,3,5-trithianyl. In certain embodiments, heterocyclic may also be optionally substituted with one or more substituents Z as described herein. The term "halogen", "'halide" or "halo" refers to fluorine, chlorine, bromine, and/or iodine.

The term "optionally substituted" is intended to mean that a group, including alkyl, alkenyl, alkynyl, cycloalkyl, aryl, aralkyl, heteroaryl, and heterocyclyl, may be substituted with one or more substituents Z, in one embodiment, one, two, three or four substituents Z, where each Z is independently selected from the group consisting of cyano, halo, OXO, nitro, C₁₋₆ alkyl, halo-C₁₋₆ alky!, C₂₋₆ alkenyl, C₂₋₆ alkynyl, C₃₋₇ cycloalkyl, C₆₋₁₄ aryl, C₇₋₁₄ aralkyl, heteroaryl, heterocyclyl, -C(O)R^{e}, -C(O)OR^{e}, -C(O)NR^{f}R^{g}, -C(NR^{e})NR^{f}R^{g}, -OR^{e}, -OC(O)R^{e}, -OC(O)OR^{e}, -OC(O)NR^{f}R^{g}. -OC(=NR^{e})NR^{f}R^{g}, -OS(O)R^{e}, -OS(O)₂R^{e}, -OS(O)NR^{f}R^{g}, -OS(O)₂NR^{f}R^{g}, -NR^{f}R^{g}, -NR^{e}C(O)R^{f}, -NR^{e}C(O)OR^{f}, -NR^{e}C(O)NR^{f}R^{g}, -NR^{e}C(=NR^{h})NR^{f}R^{g}, -NR^{e}S(O)R^{f}, -NR^{e}S(O)₂R^{f}, -NR^{c}S(O)NR^{f}R^{g}, -NR^{e}S(O)₂NR^{f}R^{g}, -SR^{e}, -S(O)R^{e}, and -S(O)₂R^{e}, and -S(O)₂NR^{f}R^{g}, wherein each R^{e}, R^{f}, R^{g}, and R^{h} is independently hydrogen, C₁₋₆ alkyl, C₂₋₆ alkenyl, C₂₋₆ alkynyl, C₃₋₇ cycloalkyl, C₆₋₁₄ aryl, C₇₋₁₄ aralkyl, heteroaryl, or heterocyclyl; or R^{f} and R^{g} together with the N atom to which they are attached form heterocyclyl.

The term "solvate" refers to a compound provided herein or a salt thereof, which further includes a stoichiometric or non-stoichiometric amount of solvent bound by non- covalent intermolecular forces. Where the solvent is water, the solvate is a hydrate.

In accordance with this disclosure, the term "comprising" is inclusive or open- ended and docs not exclude additional, unrecited elements or method steps; the term "consisting of' excludes any element, step, or ingredient not specified; and the term "consisting essentially of' excludes any element, step, or ingredient that materially changes a basic characteristic of the invention.

In some embodiments, the formulation for use according to the invention comprises at least one phospholipid, at least one nonionic surfactant, optionally suspended in a pharmaceutically acceptable medium, preferably an aqueous solution, preferably having a pH ranging from 3.5 to 9.0, preferably from 4 to 7.5. The formulation for use according to the invention may optionally contain buffers, antioxidants, preservatives, microbicides. antimicrobials, emollients, co-solvents, and/or thickeners. In some embodiments, the formulation for use according to the invention comprises a mixture of more than one phospholipid. In some embodiments, the formulation for use according to the invention consists essentially of at least one phospholipid, at least one nonionic surfactant, a pharmaceutically acceptable carrier, and optionally buffers, antioxidants, preservatives, microbicides, antimicrobials, emollients, co- solvents, and/or thickeners. In some embodiments, the formulation for use according to the invention consists of at least one phospholipid, at least one nonionic surfactant, a pharmaceutically acceptable carrier, and one or more of the following: buffers, antioxidants, preservatives, microbicides, antimicrobials, emollients, co-solvents, and thickeners.

In the sense of this disclosure, a "lipid" is any substance, which has properties like or similar to those of a fat. As a rule, it has an extended apolar group (the "chain", X) and generally also a water-soluble, polar hydrophilic part, the "head" group (Y) and has the basic Formula I:

X-Yn(**I**)

wherein n is equal to or larger than zero.

Lipids with n=0 are referred to as apolar lipids and lipids with n>1 are referred to as polar lipids. In this sense, all amphophilic substances, including, but not limited to glycerides, glyccrophospholipids, glycerophosphinolipids, glycerophosphonolipids, sulfolipids, sphingolipids, isoprenoid lipids, steroids or sterols and carbohydrate-containing lipids can generally be referred to as lipids, and are included as such in this disclosure. A list of relevant lipids and lipid related definitions is provided in EP 0 475 160 Al (see, e.g. p. 4, 1. 8 to p. 6, 1. 3) and U.S. Patent No. 6,165,500 (see, e.g., col. 6, 1. 10 to col. 7, 1. 58).

A phospholipid in various embodiments may contain (I) a moiety derived from glycerol or a sphingosine, (2) a phosphate group, and/or (3) simple organic molecule such as choline. A phospholipid as used herein may, for example, be a compound of Formula II:

R¹-CH₂-CHR²-CR³H-O-PHO₂-O-R⁴ (**II**)

wherein R¹ and R² are hydrogen, OH, an alkyl group, an aliphatic chain, an aliphatic chain derived from a fatty acid or a fatty alcohol: provided however that R¹ and R² cannot both be hydrogen, OH or a C1-C3 alkyl group,; In some embodiments R¹ and R² are independently, an aliphatic chain, most often derived from a fatty acid or a fatty alcohol; R³ generally is a hydrogen.

The OH-group of the phosphate is a hydroxyl radical or hydroxyl anion (i.e. hydroxide) form, dependent on degree of the group ionization. Furthermore, R⁴ may be a proton or a short-chain alkyl group, substituted by a tri-short-chain alkylammonium group, such as a trimethylammonium group, or an amino -substituted short-chain alkyl group, such as 2-trimethylammonium ethyl group (cholinyl) or 2-dimethylammonium short alkyl group.

A sphingophospholipid is, for example, a compound of Formula IIB:

R¹-Sphingosine-O-PHO₂-O-R⁴ (**IIB**)

wherein R¹ is a fatty-acid attached via an amide bond to the nitrogen of the sphingosine and R⁴ has the meanings given under Formula II.

A lipid preferably is a substance of formulae II or IIB, wherein R¹ and/or R² are acyl or alkyl, n-hydroxyacyl or n-hydroxyalkyl, but may also be branched, with one or more methyl groups attached at almost any point of the chain; usually, the methyl group is near the end of the chain (iso or anteiso). The radicals R¹ and R² may moreover either be saturated or unsaturated (mono-, di- or poly-unsaturated). R³ is hydrogen and R⁴ is 2-trimethylammonium ethyl (the latter corresponds to the phosphatidyl choline head group), 2-dimethylammonium ethyl, 2-methylammonium ethyl or 2-aminoethyl (corresponding to the phosphatidyl ethanolamine head group). R⁴ may also be a proton (giving phosphatidic acid), a serine (giving phosphatidylserine), a glycerol (giving phosphatidylglycerol), an inositol (giving phosphatidylinositol), or an alkylamine group (giving phosphatidylethanolamine in case of an ethylamine), if one chooses to use a naturally occurring glycerophospholipid. Otherwise, any other sufficiently polar phosphate ester, such that will form a lipid bilayer, may be considered as well for making the formulations of the disclosure.

A phospholipid is, for example, a compound of Formula IIC as described in WO2011/022707, wherein R¹ and R² are independently an acyl group, alkyl group, n-hydroxyacyl group, or n- hydroxyalkyl group, most often derived from a fatty acid or a fatty alcohol, wherein R¹ and R² may also be branched, with one or more methyl groups attached at almost any point of the chain: usually, the methyl group is near the end of the chain (iso or anteiso). wherein R¹ and R² cannot both be hydrogen, OH or a C₁-C₃ alkyl group. The radicals R¹ and R² may moreover either be saturated or unsaturated (mono-, di- or poly-unsaturated). R³ generally is a hydrogen. The OH-group of the phosphate is a hydroxyl radical or hydroxyl anion (i.e. hydroxide) form, dependent on degree of the group ionization. Furthermore. R may be a proton or a short-chain alkyl group, substituted by a tri- short-chain alkylammonium group, such as a trimethylammonium group, or an amino-substituted short-chain alkyl group, such as 2-trimethylammonium ethyl group (cholinyl) or 2-dimethylammonium short alkyl group. R⁴ may be 2-trimethylammonium ethyl (the latter corresponds to the phosphatidyl choline head group), 2-dimethylammonium ethyl, 2-methylammonium ethyl or 2-aminoethyl (corresponding to the phosphatidyl ethanolamine head group). R⁴ may also be a proton (giving phosphatidic acid), a serine (giving phosphatidylserine), a glycerol (giving phosphatidylglycerol), an inositol (giving phosphatidylinositol), or an alkylamine group (giving phosphatidylethanolamine in case of an ethylamine), if one chooses to use a naturally occurring glycerophospholipid. Otherwise, any other sufficiently polar phosphate ester, such that will form a lipid bilayer may be considered as well for making the formulations of the disclosure.

Table 1 lists preferred phospholipids in accordance with one embodiment of the disclosure.

**Table 1:**

| |
|---|
| Bechen(o)yl |
| Eruca(o)yl |
| Arachin(o)yl |
| Gadolen(o)yl |
| Arachindon(o)yl |
| Ole(o)yl |
| Stear(o)yl |
| Linol(o)yl |
| Linole(n/o)yl |
| Palmitole(o)yl |
| Palmit(o)yl |
| Myrist(o)yl |
| Laur(o)yl |
| Capr(o)yl |

The preferred lipids in the context of this disclosure are uncharged and form stable, well hydrated bilayers; phosphatidylcholines, phosphatidylethanolamine, and sphingomyelins are the most prominent representatives of such lipids. Any of those can have chains as listed in the Table 1; the ones forming fluid phase bilayers, in which lipid chains are in disordered state, being preferred.

Different negatively charged, i.e., anionic, lipids can also be incorporated into vesicular lipid bilayers. Attractive examples of such charged lipids are phosphatidylglycerols, phosphatidylinositols and, somewhat less preferred, phosphatidic acid (and its alkyl ester) or phosphatidylserine. It will be realized by anyone skilled in the art that it is less commendable to make vesicles just from the charged lipids than to use them in a combination with electro- neutral bilaycr component(s). In case of using charged lipids, buffer composition and/or pH care must selected so as to ensure the desired degree of lipid head-group ionization and/or the desired degree of electrostatic interaction between the, oppositely, charged drug and lipid molecules. Moreover, as with neutral lipids, the charged bilayer lipid components can in principle have any of the chains of the phospholipids as listed in the Table 1. The chains forming fluid phase lipid bilayers are clearly preferred, however, both due to vesicle adaptability increasing role of increasing fatty chain fluidity and due to better ability of lipids in fluid phase to mix with each other.

The fatty acid- or fatty alcohol-derived chain of a lipid is typically selected amongst the basic aliphatic chain types below:

| | | |
|---|---|---|
| Dodecanoic | cis-9-Tetradecanoic | 1 0-cis, 13-cis-Hexadecadienoic |
| Tridecanoic | cis-7-Hexadecanoic | 7-cis, 10-cis-Hexadecandienoic |
| Tetradecanoic | cis-9-Hexadecanoic | 7-cis,10-cis,13-cis-Hexadecatrienoic |
| Pentadecanoic | cis-9-Octadecanoic | 12-cis,15-cis-Octadecadienoic |
| Hexadecanoic | cis-11-Octadecanoic | trans-10,trans-12-Octadecadienoic |
| Heptadecanoic | cis-11-Eicosanoic | 9-cis,12-cis,15-cis-Octadecatrienoic |
| Octadecanoic | cis-14-Eicosanoic | 6-cis,9-cis,12-cis-Octadecatrienoic |
| Nonadecanoic | cis-13-Docosanoic | 9-cis,11-trans,13-trans-Octadecatrienoic |
| Eicosanoic | cis-15-Tetracosanoic | 8-trans, 1 0-trans, 12-cis-Octadecatrienoic |
| Heneicosanoic | trans-3-Hexadecanoic | 6,9,12,15-Octadecatetraenoic |
| Docosanoic | tans-9-Octadecanoic | 3,6,9,12-Octadecatetraenoic |
| Tricosanoic | trans-11-Octadecanoic | 3,6,9,12,15-Octadecapentaenoic |
| Tetracosanoic | | 14-cis,17-cis-Eicosadienoic |
| | | 11-cis,14-cis-Eicosadienoic |
| | | 8-cis,11-cis-14-cis-Eicosadienoic |
| | | 8-cis,11-cis-14-cis-Eicosadienoic |
| | | 5,8,11all-cis-Eicosatrienoic |
| | | 5,8,11; 14-all-cis-Eicosatrienoic |
| | | 8,11,14,17-all-cis-Eicosatetraenoic |
| | | 5,8,11,14,17-all-cis-Eicosatetraenoic |
| | | 13,16-Docosadienoic |
| | | 13,16,19-Docosadienoic |
| | | 10,13,16-Docosadienoic |
| | | 7,10,13,16-Docosadienoic |
| | | 4,7,10,13,16-Docosadienoic |
| | | 4,7,10,13,16,19-Docosadienoic |

Other double bond combinations or positions are possible as well.

Suitable fatty residues can furthermore be branched, for example, can contain a methyl group in an iso or anteiso position of the fatty acid chain, or else closer to the chain middle, as in 10-R-methyloctadecanoic acid or tuberculostearic chain Relatively important amongst branched fatty acids are also isoprenoids, many of which are derived from 3,7,11,15-tetramethylhexadec-trans-2-en-1-ol, the aliphatic alcohol moiety of chlorophyll. Examples include 5,9,13,17-tetramethyloctadecanoic acid and especially 3,7,11,15- tetramethylhexadecanoic (phytanic) and 2,6,10,14-tetramethylpentadecanoic (pristanic) acids. A good source of 4,8,12-trimethyltridecanoic acid are marine organisms. Combination of double bonds and side chains on a fatty residue are also possible.

Alternatively, suitable fatty residues may carry one or a few oxy- or cyclic groups, especially in the middle or towards the end of a chain. The most prominent amongst the later, alicyclic fatty acids, are those comprising a cyclopropane (and sometimes cyclopropcne) ring, but cyclohexyl and cycloheptyl rings can also be found and might be useful for purposes of this disclosure. 2-(D)-Hydroxy fatty acids are more ubiquitous than alicyclic fatty acids, and are also important constituents of sphingolipids. Also interesting are 15-hydroxy- hexadecanoic and 17-hydroxy-octadecanoic acids, and maybe 9-hydroxy-octadeca-trans- 10,trans-12-dienoic (dimorphecolic) and 13-hydroxy-octadeca-cis-9,trans-11-dienoic (coriolic) acid. Arguably the most prominent hydroxyl-fatty acid in current pharmaceutical use is ricinoleic acid, (D-(-)12-hydroxy-octadec-cis-9 enoic acid, which comprises up to 90% of castor oil, which is also often used in hydrogenated form. Epoxy-, mcthoxy-, and furanoid-fatty acids are of only limited practical interest in the context of this disclosure.

Generally speaking, unsaturation, branching or any other kind of derivatization of a fatty acid is best compatible with the intention of present disclosure of the site of such modification is in the middle or terminal part of a fatty acid chain. The cis-unsaturated fatty acids are also more preferable than trans-unsaturated fatty acids and the fatty radicals with fewer double bonds are preferred over those with multiple double bonds, due to oxidation sensitivity of the latter. Moreover, symmetric chain lipids are generally better suited than asymmetric chain lipids.

A preferred lipid of the Formula II is, for example, a natural phosphatidylcholine, which used to be called lecithin. It can be obtained from egg (rich in palmitic, C16:0, and oleic, C18:1, but also comprising stearic, C18:0, palmitoleic, C16:1, linolenic, C18:2, and arachidonic, C20:4(M, radicals), soybean (rich in unsaturated C18 chains, but also containing some palmitic radical, amongst a few others), coconut (rich in saturated chains), olives (rich in monounsaturated chains), saffron (safflower) and sunflowers (rich in n-6 linoleic acid), linseed (rich in n-3 linolenic acid), from whale fat (rich in monounsaturated n-3 chains), from primrose or primula (rich in n-3 chains). Preferred, natural phosphatidyl ethanolamines (used to be called cephalins) frequently originate from egg or soybeans. Preferred sphingomyelins of biological origin are typically prepared from eggs or brain tissue. Preferred phosphatidylserines also typically originate from brain material whereas phosphatidylglycerol is preferentially extracted from bacteria, such as *E. coli,* or else prepared by way of transphosphatidylation, using phospholipase D, starting with a natural phosphatidylcholine. The preferably used phosphatidylinositols are isolated from commercial soybean phospholipids or bovine liver extracts. The preferred phosphatidic acid is either extracted from any of the mentioned sources or prepared using phospholipase D from a suitable phosphatidylcholine.

Furthermore, synthetic phosphatidyl cholines (R⁴ in Formula II corresponds to 2-trimethylammonium ethyl), and R¹ and R² are aliphatic chains, as defined in the preceding paragraph with 12 to 30 carbon atoms, preferentially with 14 to 22 carbon atoms, and even more preferred with 16 to 20 carbon atoms, under the proviso that the chains must be chosen so as to ensure that the resulting ESAs comprise fluid lipid bilayers. This typically means use of relatively short saturated and of relatively longer unsaturated chains. Synthetic sphingomyelins (R⁴ in Formula IIB corresponds to 2-trimethylammonium ethyl), and R¹ is an aliphatic chain, as defined in the preceding paragraph, with 10 to 20 carbon atoms, preferentially with 10 to 14 carbon atoms per fully saturated chain and with 16-20 carbon atoms per unsaturated chain.

Synthetic phosphatidyl ethanolamines (R⁴ is 2-aminoethyl), synthetic phosphatidic acids (R⁴ is a proton) or its ester (R⁴ corresponds, for example, to a short-chain alkyl, such as methyl or ethyl), synthetic phosphatidyl serines (R⁴ i-s L- or D-serine), or synthetic phosphatidyl (poly)alcohols, such as phosphatidyl inositol, phosphatidyl glycerol (R⁴ is L- or D-glycerol) are preferred as lipids, wherein R¹ and R² are fatty residues of identical or moderately different type and length, especially such as given in the corresponding tables given before in the text. Moreover, R¹ can represent alkenyl and R² identical hydroxyalkyl groups, such as tetradecylhydroxy or hexadecylhydroxy, for example, in ditetradecyl or dihexadecylphosphatidyl choline or ethanolamine, R² can represent alkenyl and R² hydroxyacyl, such as a plasmalogen (R⁴ trimethylammonium ethyl), or R¹ can be acyl, such as lauryl, myristoyl or palmitoyl and R² can represent hydroxy as, for example, in natural or synthetic lysophosphatidyl cholines or lysophosphatidyl glycerols or lysophosphatidyl ethanolamines, such as 1-myristoyl or 1-palmitoyllysophosphatidyl choline or - phosphatidyl ethanolamine; frequently, R³ represents hydrogen.

A lipid of Formula IIB is also a suitable lipid within the sense of this disclosure. In Formula IIB, n=1, R¹ is an alkenyl group. R² is an acylamido group. R³ is hydrogen and R⁴ represents 2-trimethylammonium ethyl (choline group). Such a lipid is known under the name of sphingomyelin.

Suitable lipids furthermore are a lysophosphatidyl choline analog, such as 1- lauroyl-1,3-dihydroxypropane-3-phosphoryl choline, a monoglyceride, such as monoolein or monomyristin, a cerebroside, ceramide polyhexoside, sulfatide, sphingoplasmalogen, a ganglioside or a glyceride, which does not contain a free or esterified phosphoryl or phosphono or phosphino group in the 3 position. An example of such a glyceride is diacylglyceride or 1 -alkenyl- 1-hydroxy-2-acyl glyceride with any acyl or alkenyl groups, wherein the 3 -hydroxy group is etherified by one of the carbohydrate groups named, for example, by a galactosyl group such as a monogalactosyl glycerin.

Lipids with desirable head or chain group properties can also be formed by biochemical means, for example, by means of phospholipases (such as phospholipase Al, A2, B, C and, in particular, D), desaturases, elongases, acyl transferases, etc., from natural or synthetic precursors.

Furthermore, a suitable lipid is any lipid, which is contained in biological membranes and can be extracted with the help of apolar organic solvents, such as chloroform. Aside from the lipids already mentioned, such lipids also include, for example, steroids, such as estradiol, or sterols, such as cholesterol, beta-sitosterol, desmosterol, 7-keto-cholesterol or beta-cholestanol, fat-soluble vitamins, such as retinoids, vitamins, such as vitamin A1 or A2, vitamin E, vitamin K, such as vitamin K1 or K2 or vitamin Dl or D3, etc.

The less soluble amphiphilic components comprise or preferably comprise a synthetic lipid, such as myristoleoyl, palmitoleoyl, petroselinyl, petroselaidyl, oleoyl, elaidyl, cis- or trans-vaccenoyl, linolyl, linolenyl, linolaidyl, octadecatetraenoyl, gondoyl, eicosaenoyl, eicosadienoyl. eicosatrienoyl, arachidoyl, cis- or trans-docosaenoyl, docosadienoyl, docosatrienoyl, docosatetraenoyl, lauroyl, tridccanoyl. myristoyl, pentadccanoyl, palmitoyl, heptadecanoyl, stearoyl or nonadecanoyl, glycerophospholipid or corresponding derivatives with branched chains or a corresponding dialkyl or sphingosin derivative, glycolipid or other diacyl or dialkyl lipid.

The more soluble amphiphilic components(s) is/arc frequently derived from the less soluble components listed above and, to increase the solubility, substituted and/or complexed and/or associated with a butanoyl, pentanoyl. hcxanoyl. heptanoyl, octanoyl, nonanoyl, decanoyl or undecanoyl substituent or several, mutually independent, selected substituents or with a different material for improving the solubility.

A further suitable lipid is a diacyl- or dialkyl-glycerophosphoetha- nolamine azo polyethoxylene derivative, a didecanoylphosphatidyl choline or a diacylphosphoolligomaltobionamide.

In certain embodiments, the amount of lipid in the formulation is from about 1% to about 12%, about 1% to about 10%, about 1% to about 4%, about 4% to about 7% or about 7% to about 10% by weight. In a specific embodiment, the lipid is a phospholipid. In another specific embodiment, the phospholipid is a phosphatidylcholine.

In some embodiments, the lipid in the formulation docs not comprise an alkyl-lysophospholipid. In some embodiments, the lipid in the formulation does not comprise a polyeneylphosphatidylcholine.

The term "surfactant" has its usual meaning. A list of relevant surfactants and surfactant related definitions is provided in EP 0 475 160 A1 (see, e.g., p. 6, 1. 5 to p.14. 1.17) and U.S. Pat. No. 6,165,500 (see, e g., col. 7, 1. 60 to col. 19, 1. 64) and in appropriate surfactant or pharmaceutical Handbooks, such as Handbook of Industrial Surfactants or US Pharmacopoeia, Pharm. Eu. In some embodiments, the surfactants are those described in Tables 1-18 of U.S. Patent Application Publication No. 2002/0012680 A1 . published January 31, 2002. The following list therefore only offers a selection, which is by no means complete or exclusive, of several surfactant classes that are particularly common or useful in conjunction with present patent application. Preferred surfactants to be used in accordance with the disclosure include those with an HLB greater than 12. The list includes ionized long-chain fatty acids or long chain fatty alcohols, long chain fatty ammonium salts, such as alkyl- or alkenoyl-trimethyl-, -dimethyl- and - methyl-ammonium salts, alkyl- or alkenoyl-sulphate salts, long fatty chain dimethyl- aminoxides, such as alkyl- or alkenoyl-dimethyl-aminoxides, long fatty chain, for example alkanoyl, dimethyl-aminoxides and especially dodecyl dimethyl-aminoxide, long fatty chain, for example alkyl-N-methylglucamide- s and alkanoyl-N-methylglucamides. such as MEGA-8, MEGA-9 and MEGA-IO, N-long fatty chain-N,N-dimethylglycines, for example N-alkyl- N,N-dimethylglycines, 3 -(long fatty chain-dimethylammonio)-alkane- sulphonates, for example 3-(acyidimethylammonio)-alkanesulphonatcs, long fatty chain derivatives of sulphosuccinate salts, such as bis(2-ethylalkyl) sulphosuccinate salts, long fatty chain-sulphobetaines, for example acyl-sulphobetaines, long fatty chain betaines, such as EMPIGEN BB or ZWITTERGENT-3-16, -3-14, -3-12, -3-10, or -3-8, or polyethylcnglycol- acylphenyl ethers, especially nonaethylen-glycol-octyl- phenyl ether, polyethylene-long fatty chain-ethers, especially polyethylene-acyl ethers, such as nonaethylen-decyl ether, nonaethylen-dodecyl ether or octaethylene-dodecyl ether, polyethyleneglycol-isoacyl ethers, such as octaethyleneglycol-isotridecyl ether, polyethyleneglycol-sorbitane-long fatty chain esters, for example polyethyleneglycol-sorbitane-acyl esters and especially polyoxyethylene- monolaurate (e.g. polysorbate 20 or Tween 20), polyoxyethylene-sorbitan-monooleate (e.g. polysorbate 80 or Tween 80), polyoxyethylene-sorbitan-monolauroleylate, polyoxyethylene - sorbitan-monopetroselinate, polyoxyethylene -sorbitan- monoelaidate, polyoxyethylene - sorbitan-myristoleylate, polyoxyethylene -sorbitan-palmitoleinylate, polyoxyethylene-sorbitan-p- etroselinylate, polyhydroxyethylene-long fatty chain ethers, for example polyhydroxyethylene-acyl ethers, such as polyhydroxyethylene-lauryl ethers, polyhydroxyethylene-myristoyl ethers, polyhydroxyethylene-cetylst- earyl, polyhyd roxyethylene-palmityl ethers, polyhydroxyethylene-oleoyl ethers, polyhydroxyethylene- palmitoleoyl ethers, polyhydroxyethylene-lino- leyl, polyhydroxyethylen-4, or 6, or 8, or 10, or 12-lauryl, miristoyl, palmitoyl, palmitoleyl, oleoyl or linoeyl ethers (Brij series), or in the corresponding esters, polyhydroxyethylen-laurate, -myristate, -palmitate, -stearate or -oleate, especially polyhydroxyethylen-8-stearate (Myrj 45) and polyhydroxyethylen-8-oleate, polyethoxylated castor oil 40 (Cremophor EL), sorbitane-mono long fatty chain, for example alkylate (Arlacel or Span series), especially as sorbitane-monolaurate (Arlacel 20, Span 20), long fatty chain, for example acyl-N-methylglucamides, alkanoyl-N-methylglucamides, especially decanoyl-N-methylglucamide, dodecanoyl-N-methylglucamide, long fatty chain sulphates, for example alkyl-sulphates, alkyl sulphate salts, such as lauryl-sulphate (SDS), oleoyl-sulphate: long fatty chain thioglucosides, such as alkylthioglucosides and especially heptyl-, octyl- and nonyl-beta-D-thioglucopyranoside; long fatty chain derivatives of various carbohydrates, such as pentoses, hcxoses and disaccharidcs, especially alkyl-glucosides and maltosides, such as hexyl-, heptyl-, octyl-, nonyl- and decyl-beta-D-glucopyranoside or D- maltopyranosidc; further a salt, especially a sodium salt, of cholate, deoxycholate, glycocholate, glycodcoxycholate, taurodeoxycholate, taurocholate, a fatty acid salt, especially oleate, elaidate, linoleate, laurate, or myristate, most often in sodium form, lysophospholipids, n-octadecylene-glycerophosphatidic acid, octadecylene- phosphorylglycerol, octadecylene-phosphorylserine, n-long fatty chain-glycero-phosphatidic acids, such as n-acyl-glycero-phosphatidic acids, especially lauryl glycero-phosphatidic acids, oleoyl-glycero-phosphatidic acid, n-long fatty chain-phosphoryl glycerol, such as n- acyl-phosphorylglycerol, especially lauryl-, myristoyl-, oleoyl- or palmitoeloyl- phosphorylglycerol, n-long fatty chain-phosphorylserine, such as n-acyl-phosphorylserine, especially lauryl-, myristoyl-, oleoyl- or palmitoeloyl-phosphorylserine, n-tetradecyl-glycero- phosphatidic acid, n-tetradecyl-phosphorylglycerol, n-tetradecyl-phosphorylserine, corresponding-, elaidoyl-, vaccenyl-lysophospholipids, corresponding short-chain phospholipids, as well as all surface active and thus membrane destabilising polypeptides. Surfactant chains are typically chosen to be in a fluid state or at least to be compatible with the maintenance of fluid-chain state in carrier aggregates.

In certain embodiments, the surfactant is a nonionic surfactant. The surfactant may be present in the formulation in about 0.1% to about 5.0%, about 0.2% to 10%, about 1% to about 10%, about 1% to about 7% or about 2% to 5% by weight. In certain embodiments, the nonionic surfactant is selected from the group consisting of: polyoxyethylene sorbitans (polysorbate surfactants), polyhydroxyethylene stearates or polyhydroxyethylene laurylethers (Brij surfactants). In a specific embodiment, the surfactant is a polyoxyethylene-sorbitan- monooleate (e.g. polysorbate 80 or Tween 80) or Tween 20, 40 or 60. In certain embodiments, the polysorbate can have any chain with 12 to 20 carbon atoms. In certain embodiments, the polysorbate is fluid in the formulation, which may contain one or more double bonds, branching, or cyclo-groups.

In some embodiments, the formulations described herein comprise only one lipid and only one surfactant. In other embodiments, the formulations comprise more than one lipid and only one surfactant, e.g., two, three, four, or more lipids and one surfactant. In other embodiments, the formulations comprise only one lipid and more than one surfactant, e.g., two, three, four, or more surfactants and one lipid. In other embodiments, the formulations comprise more than one lipid and more than one surfactant, e.g., two, three, four, or more lipids and two, three, four, or more surfactants.

The formulations described herein may have a range of lipid to surfactant ratios. The ratios may be expressed in terms of molar terms (mol lipid/mol surfactant). The molar ratio of lipid to surfactant in the formulations may be from about 1:3 to about 30:1, from about 1:2 to about 30:1, from about 1:1 to about 30:1, from about 2:1 to about 20:1, from about 5:1 to about 30:1, from about 10:1 to about 30:1, from about 15: 1 to about 30:1, or from about 20:1 to about 30:1. In certain embodiments, the molar ratio of lipid to surfactant in the formulations may be from about 1:2 to about 10:1. In certain embodiments, the ratio is from about 1:1 to about 2:1, from about 2:1 to about 3:1, from about 3:1 to about 4:1. from about 4:1 to about 5:1 or from about 5:1 to about 10:1. In certain embodiments, the molar ratio is from about 10.1 to about 30:1, from about 10:1 to about 20:1, from about 10:1 to about 25:1, and from about 20:1 to about 25:1. In specific embodiments, the lipid to surfactant ratio is about 1.0:1.0, about 1.25:1.0, about 1.5/1.0, about 1.75/1.0, about 2.0/1.0, about 2.5/1.0, about 3.0/1.0 or about 4.0/1.0. The formulations may also have varying amounts of total amount of the following components: lipid and surfactant combined (TA). The TA amount may be stated in terms of weight percent of the total composition. In one embodiment, the TA is from about 1% to about 40%, about 5% to about 30%, about 7.5% to about 15%, about 6% to about 14%, about 8% to about 12%,. about 5% to about 10%, about 10% to about 20% or about 20% to about 30%. In specific embodiments, the TA is 6%, 8%, 9%, 10%, 12%, 14%, 15% or 20%.

Selected ranges for total lipid amounts and lipid/surfactant ratios (mol/mol) for the formulations of the invention are described in the Table below:

**Table 2: Total Amount and Lipid to Surfactant Ratios**

| **TA (and surfactant) (%)** | **Lipid/Surfactant (mol/mol)** |
|---|---|
| 5 to 10 | 1.0 to 1.25 |
| 5 to 10 | 1.25 to 1.72 |
| 5 to 10 | 1.75 to 2.25 |
| 5 to 10 | 2.25 to 3.00 |
| 5 to 10 | 3.00 to 4.00 |
| 5 to 10 | 4.00 to 8.00 |
| 5 to 10 | 10.00 to 13.00 |
| 5 to 10 | 15.00 to 20.00 |
| 5 to 10 | 20.00 to 22.00 |
| 5 to 10 | 22.00 to 25.00 |
| 10 to 20 | 1.0 to 1.25 |
| 10 to 20 | 1.25 to 1.75 |
| 10 to 20 | 1.25 to 1.75 |
| 10 to 20 | 2.25 to 3.00 |
| 10 to 20 | 3.00 to 4.00 |
| 10 to 20 | 4.00 to 8.00 |
| 10 to 20 | 10.00 to 13.00 |
| 10 to 20 | 15.00 to 20.00 |
| 10 to 20 | 20.00 to 22.00 |
| 10 to 20 | 22.00 to 25.00 |

The formulations for use according to the invention do not comprise a pharmaceutically active agent that has received marketing or regulatory approval in any country for the treatment of rosacea.

The formulations for use according to the invention may optionally contain one or more of the following ingredients: co-solvents, chelators, buffers, antioxidants, preservatives, microbicides, emollients, humectants, lubricants and thickeners.

Preferred amounts of optional components are described as follows.

| | Molar (M) or | Rel w%^{∗} |
|---|---|---|
| Antioxidant: | | |
| *Primary:* | | |
| Butylated hydroxyanisole, BHA | | 0.1-8 |
| Butylated hydroxytoluene BHT | | 0.1-4 |
| Thymol | | 0.1-1 |
| Metabisulphite | 1-5mM | |
| Bisulsphite | 1-5mM | |
| Thiourea (MW=76.12) | 1-10mM | |
| Monothioglycerol (MW=108.16) | 1-20mM | |
| Propyl gallate (MW=212.2) | | 0.02-0.2 |
| Ascorbate (MW=175.3⁺ ion) | 1-10mM | |
| Palmityl-ascorbate | | 0.01-1 |
| Tocopherol-PEG | | 0.5-5 |
| *Secondary* (chelator) | | |
| EDTA (MW=292) | 1-10mM | |
| EGTA (MW=380.35) | 1-10mM | |
| Desferal (MW=656.79) | 0.1-5mM | |
| *Buffer* | | |
| Acetate | 30-150mM | |
| Phosphate | 10-50mM | |
| Triethanolamine | 30-150mM | |

| | | |
|---|---|---|
| ^{∗} as a percentage of total lipid quantity | | |

The formulations for use according to the invention may include a buffer to adjust the pH of the aqueous solution to a range from pH 3.5 to pH 9, pH 4 to pH 7.5, or pH 6 to pH 7. Examples of buffers include, but are not limited to. acetate buffers, lactate buffers, phosphate buffers, and propionate buffers.

The formulations for use according to the invention are typically formulated in aqueous media. The formulations may be formulated with or without co-solvents, such as lower alcohols.

A "microbicide" or "antimicrobial'" agent is commonly added to reduce the bacterial count in pharmaceutical formulations. Some examples of microbicides are short chain alcohols, including ethyl and isopropyl alcohol, chlorbutanol, benzyl alcohol, chlorbenzyl alcohol, dichlorbenzylalcohol, hexachlorophene; phenolic compounds, such as cresol, 4-chloro-m-cresol, p-chloro-m-xylenol. dichlorophene, hexachlorophene, povidon- iodine; parabenes. especially alkyl-parabenes, such as methyl-, ethyl-, propyl-, or butyl- paraben, benzyl paraben; acids, such as sorbic acid, benzoic acid and their salts; quaternary ammonium compounds, such as alkonium salts, e.g., a bromide, benzalkonium salts, such as a chloride or a bromide, cetrimonium salts, e.g., a bromide, phenoalkecinium salts, such as phenododecinium bromide, cetylpyridinium chloride and other salts; furthermore, mercurial compounds, such as phenylmercuric acetate, borate, or nitrate, thiomersal, chlorhexidine or its gluconate, or any antibiotically active compounds of biological origin, or any suitable mixture thereof.

Examples of "antioxidants" are butylated hydroxyanisol (BHA), butylated hydroxytoluene (BHT) and di-tert-butylphenol (LY178002, LY256548, HWA-131, BF-389, CI-986, PD-127443, E-51 or 19, BI-L-239XX, etc.), tertiary butylhydroquinone (TBHQ), propyl gallate (PG), l-O-hexyl-2,3,5-trimethylhydroquinone (HTHQ); aromatic amines (diphenylamine, p-alkylthio-o-anisidine, ethylenediamine derivatives, carbazol, tetrahydroindenoindol); phenols and phenolic acids (guaiacol, hydroquinone, vanillin, gallic acids and their esters, protocatechuic acid, quinic acid, syringic acid, ellagic acid, salicylic acid, nordihydroguaiaretic acid (NDGA), eugenol); tocopherols (including tocopherols (alpha, beta, gamma, delta) and their derivatives, such as tocopheryl-acylate (e g. - acetate. - laurate. myristate, -palmitate, -oleate, -linoleate. etc., or an y other suitable tocopheryl- lipoate). tocopheryl-POE-succinate; trolox and corresponding amide and thiocarboxamide analogues; ascorbic acid and its salts, isoascorbate, (2 or 3 or 6)-o-alkylascorbic acids, ascorbyl esters (e.g., 6-o-lauroyl, myristoyl, palmitoyl-, oleoyl, or linoleoyl-L-ascorbic acid, etc.). Also useful are the preferentially oxidised compounds, such as sodium bisulphite, sodium metabisulphite, thiourea; chellating agents, such as EDTA, GDTA, desferral: miscellaneous endogenous defence systems, such as transferrin, lactoferrin, ferritin, cearuloplasmin, haptoglobion, heamopexin, albumin, glucose, ubiquinol-10); enzymatic antioxidants, such as superoxide dismutase and metal complexes with a similar activity, including catalase, glutathione peroxidase, and less complex molecules, such as beta- carotene, bilirubin, uric acid; flavonoids (flavones, flavonols, flavonones, flavanonals, chacones, anthocyanins). N-acetylcystein, mesna. glutathione, thiohistidine derivatives, triazoles; tannines, cinnamic acid, hydroxycinnamatic acids and their esters (coumaric acids and esters, caffeic acid and their esters, ferulic acid, (iso-) chlorogenic acid, sinapic acid); spice extracts (e.g., from clove, cinnamon, sage, rosemary, mace, oregano, allspice, nutmeg); carnosic acid, carnosol, carsolic acid; rosmarinic acid, rosmaridiphenol, gentisic acid, ferulic acid; oat flour extracts, such as avenanthramide 1 and 2; thioethers, dithioethers, sulphoxides, tetralkylthiuram disulphides; phytic acid, steroid derivatives (e.g., U74006F); tryptophan metabolites (e.g., 3-hydroxykynurenine, 3-hydroxyanthranilic acid), and organochalcogenides.

"Thickeners" are used to increase the viscosity of pharmaceutical formulations to and may be selected from selected from pharmaceutically acceptable hydrophilic polymers, such as partially etherified cellulose derivatives, comprising carboxym ethyl-, hydroxyethyl-, hydroxypropyl-, hydroxypropylmethyl- or methyl-cellulose; completely synthetic hydrophilic polymers comprising polyacrylates, polymethacrylatcs, poly(hydroxyethyl)-, poly(hydroxypropyl)-, poly(hydroxypropylmethyl)methacrylate, polyacrylonitrile, methallyl- sulphonate, polyethylenes, polyoxiethylenes, polyethylene glycols, polyethylene glycol- lactide, polyethylene glycol-diacrylate, polyvinylpyrrolidone, polyvinyl alcohols, poly(propylmethacrylamide), poly(propylene fumarate-co-ethylene glycol), poloxamers, polyaspartamide. (hydrazine cross-linked) hyaluronic acid, silicone; natural gums comprising alginates, carrageenan, guar-gum, gelatine, tragacanth, (amidated) pectin, xanthan, chitosan collagen, agarose; mixtures and further derivatives or co-polymers thereof and/or other pharmaceutically, or at least biologically, acceptable polymers.

The formulations for use according to the present invention may also comprise a polar liquid medium. The formulations for use according to the invention may be administered in an aqueous medium. The formulations for use according to the present invention may be in the form of a solution, suspension, emulsion, cream, lotion, ointment, gel, spray, film forming solution or lacquer.

Described herein is the use of a vesicular formulation as described above for the preparation of a pharmaceutical composition for the treatment of disorders related to fatty acid deficiencies, fatty acid metabolism, hypertriglyceridemia and hypercholesterolemia. In some embodiments, the vesicular formulation or pharmaceutical composition comprising at least one phospholipid and one surfactant for the treatment of disorders related to fatty acid deficiencies, fatty acid metabolism, hypertriglyceridemia and hypercholesterolemia wherein the formulation or pharmaceutical composition is formulated for subcutaneous, topical or intravenous delivery. The surfactant may be nonionic.

While not to be limited to any mechanism of action or any theory, the formulations for use according to the invention may form vesicles or ESAs characterized by their adaptability, deformability, or penetrability. Vesicles for use according to this invention as described in both WO 2010/140061 and in WO 2011/022707.

### EXAMPLES

The present invention is described with reference to the following examples and figures in which:

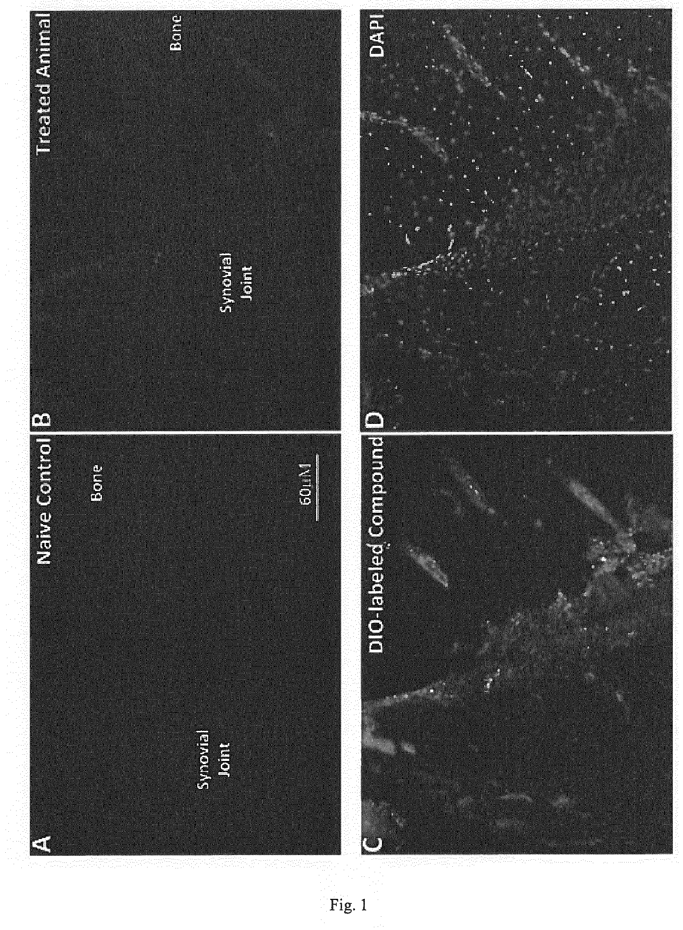

Figure 1 shows the results of Example 2 wherein the panels are as follows
   a. Control subject, untreated. There is an absence of fluorescing components
   b. Treated subject in which the image is irradiated to display the (red) fluorescent vesicles which are observed to be localised on cartilage surfaces and absent from the surrounding tissue and synovial fluid
   c. Identical section to b) but in which the vesicles are alternatively viewed in black and white (grey) imaging
   d. Identical section to b) + c) following treatment with DAPI stain to reveal the nuclei of cells. Bone and cartilage can be identified by the concentration of cells, synovial fluid has only limited cellular content;
Figure 2 shows a schematic diagram showing from where in the knee joint the section of Figure 1 was taken;
Figure 3 shows the mean percentage change from baseline in WOMAC Stiffness Subscale scores (with adjustment for analgesics) after 3 months of therapy (n=753, ITT population);
Figure 4 shows the mean percentage change from baseline in WOMAC Function Subscale scores (with adjustment for analgesics) after 3 months of therapy (n=943, ITT population);
Figure 5 shows the mean percentage change from baseline in WOMAC Pain, Function and Stiffness Subscale scores (with adjustment for analgesics) after 3 months of therapy with Formulation X (n=472) and celecoxib (n=233) (ITT population) in study CL-033-III-03;
Figure 6 shows the average VAS scores for pain and stiffness over 3 weeks of treatment with Formulation X;

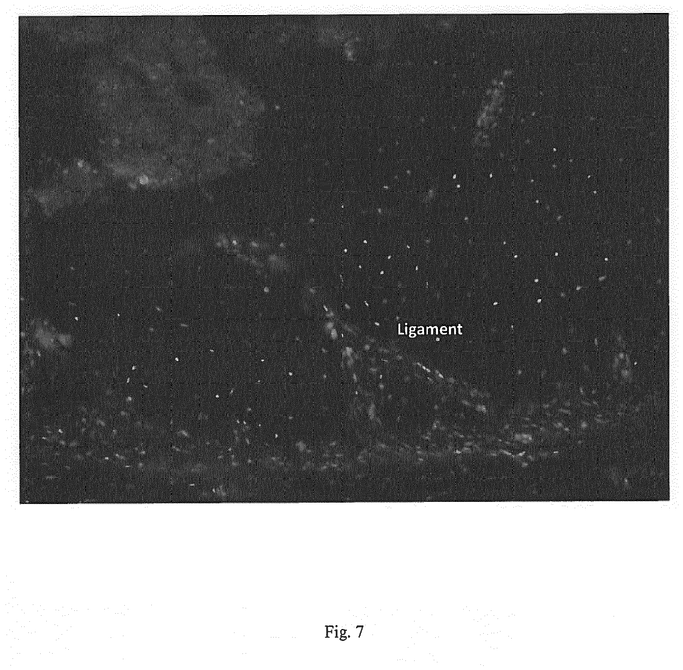

Figure 7 shows an Image of DIO labelled vesicles penetrating and occupying collagenous material, namely ligamentous structures taken from rat knee joints; and

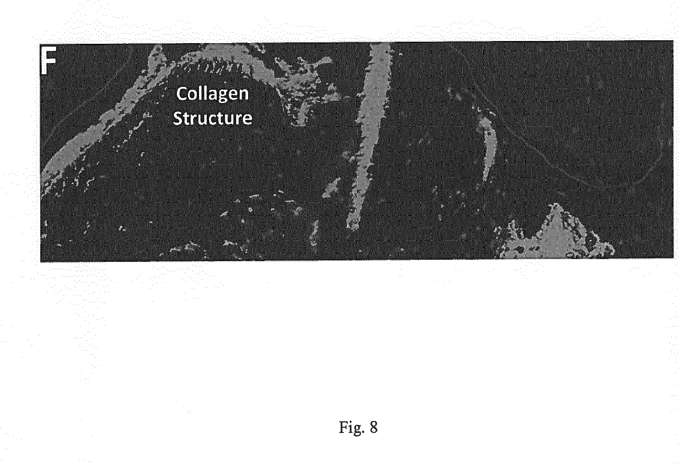

Figure 8 shows an image of labelled vesicles occupying collagenous structures taken from a rat knee joint. Bone is shown outlined in red.

### Example 1: Example Formulations

The following exemplary formulations for topical application may be prepared by the following procedure:
1. Organic phase production, which contains all lipophilic excipicnts
   The organic phase is produced by weighing the lipid, the surfactant, any additional lipophilic excipients into suitable containers followed by mixing these components into anoptically isotropic phase which appears as a clear solution. During mixing, the organic phase will be heated up, but temperature must not rise above 45°C.
2. Aqueous phase production
   The aqueous phase is prepared by weighing the non-lipophilic components and water, which serves as solvent, into suitable containers and then mixing these components into a clear solution. During mixing, the temperature will be elevated to 40°C.
3. Production of a concentrated intermediate by combination of both phases
   The isotropic organic phase and the clear aqueous phase are combined under stirring in a suitable vessel. Before and during the combination the temperature of both phases must be kept between 35°C and 45°C. The resulting intermediate is homogenised mechanically at 40°C. Before starting homogenisation, the pressure in the production vessel is lowered to - 0.08 MPa. The desired average carrier size is typically reached after 10 minutes of homogenisation.

Three process parameters must be controlled carefully during the production of the concentrated intermediate: temperature, homogeniser circulation velocity, and overall processing time.

4. Production of the final bulk product by mixing the concentrated intermediate with dilution buffer.

The concentrated intermediate is diluted with the dilution buffer to the intended final concentration. The mixture is carefully stirred in the mixing vessel at 20°C to homogeneity.

Table 2 describes the amounts of surfactant and lipids, and other excipients in the transfersomes formulations, described in terms of the percent of the total amount of formulation.

### Example Formulation 1

Formulation 1 comprises sphingomyelin (brain) (47.944 mg/g) as a lipid, Tween 80 (42.05mg/g) as a surfactant, lactate buffer (pH 4). benzyl alcohol or paraben (5.000 mg/g) as an antimicrobial agent, BHT (0.200 mg/g) and sodium metabisulfite (.0500 mg/g) as antioxidants, glycerol (30.000 mg/g), EDTA (3.000 mg/g) as a chelating agent, and ethanol (30.000 mg/g).

### Example Formulation 2

Formulation 2 comprises sphingomyelin (brain) (53.750 mg/g) as a lipid, Tween 80 (31.250 mg/g) as a surfactant, lactate (pH 4) buffer, benzyl alcohol or paraben (5.000 mg/g) as an antimicrobial agent, BHT (0.200 mg/g) and sodium metabisulfite (0.500 mg/g) as antioxidants, glycerol (30.000 mg/g), EDTA (3.000 mg/g) as a chelating agent, and ethanol (15.000 mg/g).

### Example Formulation 3

Formulation 3 comprises sphingomyelin (brain) (90.561 mg/g) as a lipid, Tween 80 (79.439 mg/g) as a surfactant, lactate (pH 4) buffer, benzyl alcohol or paraben (5.000 mg/g) as an antimicrobial agent, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 4

Formulation 4 comprises sphingomyelin (brain) (47.944 mg/g) as a lipid, Tween 80 (42.056 mg/g) as a surfactant, lactate (pH 5) buffer, benzyl alcohol or paraben (5.000 mg/g) as an antimicrobial agent, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 5

Formulation 5 comprises sphingomyelin lauroyl (50.607 mg/g) as a lipid, Brij 98 (44.393 mg/g) as a surfactant, acetate (pH 5) buffer, benzyl alcohol or paraben (5.000 mg/g) as an antimicrobial agent, BHT (0.200 mg/g) and sodium metabisulfite (0.500 mg/g) as antioxidants, EDTA (3.000 mg/g) as a chelating agent, and ethanol (10.000 mg/g).

### Example Formulation 6

Formulation 6 comprises sphingomyelin lauroyl (90.561 mg/g) as a lipid, Brij 98 (79.439 mg/g) as a surfactant, acetate (pH 5) buffer, benzyl alcohol or paraben (5.000 mg/g) as an antimicrobial agent, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 7

Formulation 7 comprises sphingomyelin lauroyl (49.276 mg/g) as a lipid, Brij 98 (79.439 mg/g) as a surfactant, acetate (pH 6.5) buffer, benzyl alcohol or paraben (5.000 mg/g) as an antimicrobial agent, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 8

Formulation 8 comprises phosphatidyl choline and phosphatidyl glycerol (53.750 mg/g) as a lipid, Brij 98 (31.250 mg/g) as a surfactant, phosphate (pH 6.5) buffer, benzyl alcohol or paraben (5.000 mg/g) as an antimicrobial agent, HTHQ (0.200 mg/g) as an antioxidant, glycerol (30.000 mg/g), and EDTA (3.000 mg/g) as a chelating agent.

### Example Formulation 9

Formulation 9 comprises phosphatidyl choline and phosphatidyl glycerol (90.561 mg/g) as a lipid, Brij 98 (79.439 mg/g) as a surfactant, phosphate (pH 6.5) buffer, benzyl alcohol or paraben (5.000 mg/g) as an antimicrobial agent, HTHQ (0.200 mg/g) as an antioxidant, glycerol (30.000 mg/g), EDT[Lambda] (3.000 mg/g) as a chelating agent, and ethanol (30.000 mg/g).

### Example Formulation 10

Formulation 10 comprises phosphatidyl choline and phosphatidyl glycerol (41.351 mg/g) as a lipid. Brij 98 (48.649 mg/g) as a surfactant, phosphate (pH 4) buffer, benz>1 alcohol or paraben (5.000 mg/g) as an antimicrobial agent, HTIIQ (0.200 mg/g) as an antioxidant, glycerol (30.000 mg/g), EDTA (3.000 mg/g) as a chelating agent, and ethanol (30.000 mg/g).

### Example Formulation 11

Formulation 1 1 comprises phosphatidyl choline and phosphatidyl glycerol (47.882 mg/g) as a lipid. Brij 98 (37.1 18 mg/g) as a surfactant, phosphate (pH 4) buffer, benzyl alcohol or paraben (5.000 mg/g) as an antimicrobial agent, HTHQ (0.200 mg/g) as an antioxidant, glycerol, EUTA (3,000 mg/g) as a chelating agent, and ethanol (30.000 mg/g).

### Example Formulation 12

Formulation 12 comprises phosphatidyl choline and phosphatidyl glycerol (95.764 mg/g) as a lipid, Brij 98 (74.236 mg/g) as a surfactant, phosphate (pi I 4) buffer, benzyl alcohol or paraben (5.000 mg/g) as an antimicrobial agent, HTHQ (0.200 mg/g) as an antioxidant, glycerol (30.000 mg/g), EDTA (3.000 mg/g) as a chelating agent, and ethanol (30.000 mg/g).

### Example Formulation 13

Formulation 13 comprises phosphatidyl choline and phosphatidylinositol (66.676 mg/g) as a lipid, Span 20 (24.324 mg/g) as a surfactant, acetate (pH 5) buffer, benzyl alcohol or paraben (5.000 mg/g), I ITI IQ (0.200 mg/g) as an antioxidant, EDTA (3.000 mg/g) as a chelating agent, and ethanol (25.000 mg/g).

### Example Formulation 14

Formulation 14 comprises phosphatidyl choline and phosphatidylinositol (62.027 mg/g) as a lipid, Span 20 (22.973 mg/g) as a surfactant, acetate (pH 5) buffer, benzyl alcohol or paraben (5.000 mg/g) as an antimicrobial agent, HTHQ (0.200 mg/g) as an antioxidant, EDTA (3.000 mg/g) as a chelating agent, and ethanol (30.000 mg/g).

### Example Formulation 15

Formulation 15 comprises phosphatidyl choline and phosphatidylinositol (124.054 mg/g) as a lipid, Span 20 (45.946 mg/g) as a surfactant, acetate (pH 5) buffer, benzyl alcohol or paraben (5.000 mg/g) as an antimicrobial agent, HTHQ (0.200 mg/g) as an antioxidant, glycerol (30.000 mg/g), and EDTA (3.000 mg/g) as a chelating agent, and ethanol (36.510 mg/g).

### Example Formulation 16

Formulation 16 comprises phosphatidyl choline and phosphatidylinositol (62.687 mg/g) as a lipid, Span 20 (32,313 mg/g) as a surfactant, acetate (pH 6.5) buffer, benzyl alcohol or paraben (5.000 mg/g) as an antimicrobial agent, HTHQ (0.200 mg/g) as an antioxidant, glycerol (30.000 mg/g), EDTA (3.000 mg/g) as a chelating agent.

### Example Formulation 17

Formulation 17 comprises phosphatidyl choline and phosphatidic acid (41.853 mg/g) as a lipid, Tween 80 (43.147 mg/g) as a surfactant, phosphate (pH 6.5) buffer, benzyl alcohol or paraben (5.000 mg/g) as an antimicrobial agent, BHT (0.200 mg/g) as an antioxidant, glycerol (30.000 mg/g), EDTA (3.000 mg/g), and ethanol (30.000 mg/g).

### Example Formulation 18

Formulation 18 comprises phosphatidyl choline and phosphatidic acid (95.764 mg/g) as a lipid, Tween 80 (74.236 mg/g) as a surfactant, phosphate (pH 6.5) buffer, benzyl alcohol or paraben (5.000 mg/g) as an antimicrobial agent, BHT (0.200 mg/g) as an antioxidant, EDTA (3.000 mg/g), and ethanol (30.000 mg/g).

### Example Formulation 19

Formulation 19 comprises phosphatidyl choline and phosphatidic acid (47.882 mg/g) as a lipid, Brij 98 and Tween 80 (37.118 mg/g) as a surfactant, phosphate (pH 6.5) buffer, benzyl alcohol or paraben (5.000 mg/g) as an antimicrobial agent, BHT (0.200 mg/g) as an antioxidant, and EDTA (3.000 mg/g).

### Example Formulation 20

Formulation 20 comprises phosphatidyl choline and phosphatidic acid (45.000 mg/g) as a lipid, Span 20 and Tween 80 (45.000 mg/g) as a surfactant, phosphate (pH 6.5) buffer, benzyl alcohol or paraben (5.000 mg/g) as an antimicrobial agent, BHT (0.200 mg/g) as an antioxidant, and EDTA (1.000 mg/g).

### Example Formulation 21

Formulation 21 comprises phosphatidyl choline (31.935 mg/g) as a lipid, cremophor and Span 20 (58.065 mg/g) as a surfactant, lactate (pH 5) buffer, thimerosal (5.000 mg/g) as an antimicrobial agent, BHA (0,200 mg/g) as an antioxidant, glycerol (30.000 mg/g), EDTA (3.000 mg/g) as a chelating agent, and ethanol (15.000 mg/g).

### Example Formulation 22

Formulation 22 comprises phosphatidyl choline (42.500 mg/g ) as a lipid, cremophor and Tween 80 (42.500 mg/g ) as a surfactant, lactate (pH 6,5) buffer, thimerosal (5.000 mg/g) as an antimicrobial agent, BHA (0.200 mg/g) as an antioxidant, glycerol (30.000 mg/g). and EDTA (3.000 mg/g) as a chelating agent.

### Example Formulation 23

Formulation 23 comprises phosphatidyl choline (38.276 mg/g) as a lipid, cremophor (51.724 mg/g) as a surfactant, lactate (pH 4) buffer, thimerosal (5.000 mg/g) as an antimicrobial agent. BHA (0.200 mg/g) as an antioxidant, EDTA (3.000 mg/g) as a chelating agent, and ethanol (36.510 mg/g).

### Example Formulation 24

Formulation 24 comprises phosphatidyl choline (42.500 mg/g ) as a lipid, cremophor (42.500 mg/g) as a surfactant, lactate (pH 4) buffer, thimerosal (5.000 mg/g) as an antimicrobial agent, BHA (0.200 mg/g) as an antioxidant, EDTA (3.000 mg/g) as a chelating agent, and ethanol (15.000 mg/g).

### Example Formulation 25

Formulation 25 comprises phosphatidyl choline (85.000 mg/g) as a lipid, cremophor (85.000 mg/g) as a surfactant, lactate (pH 4) buffer, thimerosal (5.000 mg/g) as an antimicrobial agent, BHA (0.200 mg/g) as an antioxidant, EDTA (3.000 mg/g) as a chelating agent, and ethanol (30.000 mg/g).

### Example Formulation 26

Formulation 26 comprises phosphatidyl choline (38.276 mg/g) as a lipid, cremophor (51.276 mg/g) as a surfactant, lactate (pH 5) buffer, thimerosal (5.000 mg/g) as an antimicrobial agent, BHA (0.200 mg/g) as an antioxidant, and EDTA (1.000 mg/g) as a chelating agent.

### Example Formulation 27

Formulation 27 comprises phosphatidyl choline (36.429 mg/g) as a lipid, cremophor (48.571 mg/g) as a surfactant, lactate (pH 5) buffer, thimerosal (5.000 mg/g) as an antimicrobial agent, BHA (0.200 mg/g) as an antioxidant, EDTA (3.000 mg/g) as a chelating agent, and ethanol (30.000 mg/g).

### Example Formulation 28

Formulation 28 comprises phosphatidyl choline (72.299 mg/g) as a lipid, cremophor (97,701 mg/g) as a surfactant, lactate (pH 5) buffer, thimerosal (5.000 mg/g) as an antimicrobial agent, BHA (0.200 mg/g) as an antioxidant, EDTA (3.000 mg/g) as a chelating agent, and ethanol (15.000 mg/g).

### Example Formulation 29

Formulation 29 comprises phosphatidyl ethanolamine (46.250 mg/g) as a lipid, Tween 80 (46.250 mg/g) as a surfactant, phosphate (pH 6.5) buffer, thimerosal (5.000 mg/g) as an antimicrobial agent, BHT (0.200 mg/g) and sodium metabisulfite (0.500 mg/g) as an antioxidant, EDTA (3.000 mg/g) as a chelating agent, and ethanol (20.000 mg/g).

### Example Formulation 30

Formulation 30 comprises phosphatidyl ethanolamine (38.804 mg/g) as a lipid, Tween 80 (46.196 mg/g) as a surfactant, phosphate (pH 6.5) buffer, thimerosal (5.000 mg/g) as an antimicrobial agent, BHT (0.200 mg/g) and sodium metabisulfite (0.500 mg/g) as an antioxidant, glycerol (15.000 mg/g), EDTA (3.000 mg/g) as a chelating agent, and ethanol (30.000 mg/g).

### Example Formulation 31

Formulation 31 comprises phosphatidyl ethanolamine (36.667 mg/g) as a lipid, Brij 98 and Tween 80 (33.333 mg/g) as a surfactant, phosphate (pH 6.5) buffer, thimerosal (5.000 mg/g) as an antimicrobial agent, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 32

Formulation 32 comprises phosphatidyl glycerol (23.333 mg/g) as a lipid, cremophor and Brij 98 (66.667 mg/g) as a surfactant, acetate (pH 4) buffer, benzyl alcohol or paraben (5.000 mg/g) as an antimicrobial agent, BHT (0.200 mg/g) as an antioxidant, and EDTA (3.000 mg/g) as a chelating agent.

### Example Formulation 33

Formulation 33 comprises phosphatidyl glycerol (45.833 mg/g) as a lipid, Brij 98 (41.667 mg/g) as a surfactant, acetate (pH 4) buffer, benzyl alcohol or paraben (5.000 mg/g) as an antimicrobial agent, BHT (0.200 mg/g) as an antioxidant, glycerol (30.000 mg/g), and EDTA (3.000 mg/g) as a chelating agent.

Formulation 34 comprises phosphatidyl glycerol (31.957 mg/g) as a lipid, Brij 98 (38.043 mg/g) as a surfactant, acetate (pH 4) buffer, benzyl alcohol or paraben (5.000 mg/g) as an antimicrobial agent. BHT (0.200 mg/g) as an antioxidant, EDTA (3.000 mg/g) as a chelating agent, and ethanol (30.000 mg/g).

### Example Formulation 35

Formulation 35 comprises phosphatidyl glycerol (47.143 mg/g) as a lipid, Brij 98 (42.857 mg/g) as a surfactant, acetate (pH 5) buffer, benzyl alcohol or paraben (5.000 mg/g) as an antimicrobial agent, BHT (0.200 mg/g) as an antioxidant, glycerol (30.000 mg/g), EDT[Lambda] (1.000 mg/g) as a chelating agent, and ethanol (25.000 mg/g).

### Example Formulation 36

Formulation 36 comprises phosphatidyl glycerol (96.905 mg/g) as a lipid, Brij 98 (88.095 mg/g) as a surfactant, acetate (pH 5) buffer, benzyl alcohol or paraben (5.000 mg/g) as an antimicrobial agent, BHT (0.200 mg/g) as an antioxidant, glycerol (30.000 mg/g), EDTA (3.000 mg/g) as a chelating agent, and ethanol (20.000 mg/g).

### Example Formulation 37

Formulation 37 comprises phosphatidyl glycerol (31.957 mg/g) as a lipid, Brij 98 (38.043) as a surfactant, acetate (pH 5) buffer, benzyl alcohol or paraben (5.000 mg/g) as an antimicrobial agent, BHT (0.200 mg/g) as an antioxidant, EDTA (3.000 mg/g) as a chelating agent, and ethanol (30.000 mg/g).

### Example Formulation 38

Formulation 38 comprises phosphatidyl ethanolamine (35.455 mg/g) as a lipid, cremophor (54.545 mg/g) as a surfactant, phosphate (pH 6.5) buffer, benzyl alcohol or paraben (5.000 mg/g) as an antimicrobial agent, BHT (0.200 mg/g) and sodium metabisulfite (0.500 mg/g) as antioxidants, glycerol (30.000 mg/g), and EDTA (3.000 mg/g) as a chelating agent.

### Example Formulation 39

Formulation 39 comprises phosphatidyl ethanolamine (84.457 mg/g) as a lipid, cremophor (100.543 mg/g) as a surfactant, phosphate (pH 6.5) buffer, benzyl alcohol or paraben (5.000 mg/g) as an antimicrobial agent, BHT (0.200 mg/g) and sodium metabisulfite (0.500 mg/g) as antioxidants, EDTA (3.000 mg/g) as a chelating agent, and ethanol (30.000 mg/g).

### Example Formulation 40

Formulation 40 comprises phosphatidyl ethanolamine (89.048 mg/g) as a lipid, cremophor (80.952 mg/g) as a surfactant, phosphate (pH 6.5) buffer, benzyl alcohol or paraben (5.000 mg/g), BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 41

Formulation 41 comprises phosphatidyl glycerol (41.087 mg/g) as a lipid, Tween 80 (48.913 mg/g) as a surfactant, propionate (pH 4) buffer, benzyl alcohol or paraben (5.000 mg/g) as an antimicrobial agent, BHT (0.200 mg/g) and sodium metabisulfite (0.500 mg/g) as antioxidants, glycerol (30.000 mg/g), EDTA (3.000 mg/g) as a chelating agent, and ethanol (36.510 mg/g).

### Example Formulation 42

Formulation 42 comprises phosphatidyl glycerol (45.280 mg/g) as a lipid, Tween 80 (39.720 mg/g) as a surfactant, propionate (pH 4) buffer, benzyl alcohol or paraben (5.000 mg/g) as an antimicrobial agent, BHT (0.200 mg/g) and sodium metabisulfite (0.500 mg/g), and EDTA (3.000 mg/g) as a chelating agent.

### Example Formulation 43

Formulation 43 comprises phosphatidyl glycerol (107.500 mg/g) as a lipid, Tween 80 (62.500 mg/g) as a surfactant, propionate (pH 4) buffer, benzyl alcohol or paraben (5.000 mg/g) as an antimicrobial agent, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 44

Formulation 44 comprises phosphatidyl glycerol (77.243 mg/g) as a lipid, Tween 80 (67.757 mg/g) as a surfactant, propionate (pH 4) buffer, benzyl alcohol or paraben (5.000 mg/g) as an antimicrobial agent. BHT (0.200 mg/g) and sodium metabisulfite (0.500 mg/g) as antioxidants. EDTA (3,000 mg/g) as a chelating agent, and ethanol (30.000 mg/g).

### Example Formulation 45

Formulation 45 comprises phosphatidyl glycerol (45.280 mg/g) as a lipid, Tween 80 (39.720 mg/g) as a surfactant, propionate (pH 5) buffer, benzyl alcohol or paraben (5.000 mg/g) as an antimicrobial agent, BHT (0.200 mg/g) and sodium metabisulfite (0.500 mg/g) as antioxidants, EDTA (3.000 mg/g) as a chelating agent, and ethanol (30.000 mg/g).

### Example Formulation 46

Formulation 46 comprises phosphatidyl glycerol (90.561 mg/g) as a lipid, Tween 80 (79.439 mg/g) as a surfactant, propionate (pH 5) buffer, benzyl alcohol or paraben (5.000 mg/g) as an antimicrobial agent, BHT (0.200 mg/g) and sodium metabisulfite (0.500 mg/g) as antioxidants, EDTA (3.000 mg/g) as a chelating agent, and ethanol (30.000 mg/g).

### Example Formulation 47

Formulation 47 comprises phosphatidyl glycerol (47.944 mg/g) as a lipid, Tween 80 (42.056 mg/g) as a surfactant, propionate (pH 5) buffer, benzyl alcohol or paraben (5.000 mg/g) as an antimicrobial agent, BHT (0.200 mg/g) and sodium metabisulfite (0.500 mg/g) as antioxidants, EDTA (3.000 mg/g) as a chelating agent, and ethanol (10.000 mg/g).

### Example Formulation 48

Formulation 48 comprises phosphatidyl serine (50.607 mg/g) as a lipid, Brij 98 (44.393 mg/g) as a surfactant, phosphate (pH 5.5) buffer, thimerasol (5.000 mg/g) as an antimicrobial agent, BHT (0.200 mg/g) and sodium metabisulfite (0.500 mg/g) as antioxidants, glycerol (30.000 mg/g), and EDTA (1.000 mg/g) as a chelating agent.

### Example Formulation 49

Formulation 49 comprises phosphatidyl serine (107.500 mg/g) as a lipid, Brij 98 (62.500 mg/g) as a surfactant, phosphate (pH 5.5) buffer, thimerasol (5.000 mg/g) as an antimicrobial agent, BHT (0.200 mg/g) and sodium metabisulfite (0.500 mg/g) as antioxidants, glycerol (30.000 mg/g), and EDl A (3.000 mg/g) as a chelating agent.

### Example Formulation 50

Formulation 50 comprises phosphatidyl serine (47.944 mg/g) as a lipid, Brij 98 (42.056 mg/g) as a surfactant, phosphate (pH 5.5) buffer, thimerasol (5.000 mg/g) as an antimicrobial agent, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 51

Formulation 51 comprises phosphatidyl glycerol (46.364 mg/g) as a lipid, Brij 98 (38.636 mg/g) as a surfactant, acetate (pH 4) buffer, benzyl alcohol or paraben (5.000 mg/g) as an antimicrobial, BHT (0.200 mg/g) as an antioxidant, glycerol (30.000 mg/g), EDTA (3.000 mg/g) as a chelating agent, and ethanol (25.000 mg/g).

### Example Formulation 52

Formulation 52 comprises phosphatidyl glycerol (46.364 mg/g) as a lipid, Brij 98 (38.636 mg/g) as a surfactant, acetate (pH 4) buffer, benzyl alcohol or paraben (5.000 mg/g) as an antimicrobial, BHT (0.200 mg/g) as an antioxidant, EDTA (3.000 mg/g) as a chelating agent, and ethanol (20.000 mg/g).

### Example Formulation 53

Formulation 53 comprises phosphatidyl glycerol (46.098 mg/g) as a lipid, Brij 98 (43.902 mg/g) as a surfactant, acetate (pH 5) buffer, benzyl alcohol or paraben (5.000 mg/g) as an antimicrobial, BHT (0.200 mg/g) as an antioxidant, glycerol (15.000 mg/g), EDTA (3.000 mg/g) as a chelating agent, and ethanol (30.000 mg/g).

### Example Formulation 54

Formulation 54 comprises phosphatidyl glycerol (43.537 mg/g) as a lipid, Brij 98 (41.463 mg/g) as a surfactant, acetate (pH 5) buffer, benzyl alcohol or paraben (5.000 mg/g) as an antimicrobial, BHT (0.200 mg/g) as an antioxidant, glycerol (30.000 mg/g), and EDTA (3.000 mg/g) as a chelating agent.

### Example Formulation 55

Formulation 55 comprises phosphatidyl glycerol (45.000 mg/g) as a lipid, Brij 98 (45.000 mg/g) as a surfactant, acetate (pH 5) buffer, benzyl alcohol or paraben (5.000 mg/g) as an antimicrobial, BHT (0.200 mg/g) as an antioxidant, EDTA (3.000 mg/g) as a chelating agent, and ethanol (30.000 mg/g).

### Example Formulation 56

Formulation 56 comprises phosphatidyl glycerol (59.492 mg/g) as a lipid, Brij 98 (30.508 mg/g) as a surfactant, acetate (pH 6.5) buffer, benzyl alcohol or paraben (5.000 mg/g) as an antimicrobial, BHT (0.200 mg/g) as an antioxidant, glycerol (30.000 mg/g), EDTA (3.000 mg/g) as a chelating agent, and ethanol (30.000 mg/g).

### Example Formulation 57

Formulation 57 comprises phosphatidyl glycerol (39.054 mg/g) as a lipid, Brij 98 (45.946 mg/g) as a surfactant, acetate (pH 6.5) buffer, benzyl alcohol or paraben (5.000 mg/g) as an antimicrobial, BHT (0.200 mg/g) as an antioxidant, and EDTA (3.000 mg/g) as a chelating agent.

### Example Formulation 58

Formulation 58 comprises phosphatidyl glycerol (35.854 mg/g) as a lipid, Brij 98 (34.146 mg/g) as a surfactant, acetate (pH 6.5) buffer, benzyl alcohol or paraben (5.000 mg/g) as an antimicrobial, BHT (0.200 mg/g) as an antioxidant, glycerol (30.000 mg/g), and EDTA (3.000 mg/g) as a chelating agent.

### Example Formulation 59

Formulation 59 comprises phosphatidyl choline (50.000 mg/g) as a lipid, Tween 80 (40.000 mg/g) as a surfactant, phosphate (pH 6.5) buffer, benzyl alcohol or paraben (5.000 mg/g) as an antimicrobial, BHT (0.200 mg/g) and sodium metabisulf[iota]te (0.500 mg/g) as antioxidants, glycerol (30.000 mg/g), EDTA (3.000 mg/g) as a chelating agent, and ethanol (30.000 mg/g).

### Example Formulation 60

Formulation 60 comprises phosphatidyl choline (38.571 mg/g) as a lipid, Tween 80 (51.429 mg/g) as a surfactant phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g), and ethanol (30.000 mg/g).

### Example Formulation 61

Formulation 61 comprises phosphatidyl choline (41.954 mg/g) as phospholipid, Tween 80 (50.546 mg/g) as surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g), and ethanol (30.000 mg/g).

### Example Formulation 62

Formulation 62 comprises phosphatidyl choline (42.632 mg/g) as a lipid, Tween 80 (47.368 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 63

Formulation 63 comprises phosphatidyl choline (46.098 mg/g) as a lipid, Tween 80 (43.902 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 64

Formulation 64 comprises phosphatidyl choline (39.721 mg/g) as a lipid, Tween 80 (50.279 mg/g) as a surfactant, phosphate (pH 6.5) buffer, benzyl alcohol or paraben (5.000 mg/g) as an antimicrobial, BH T (0.200 mg/g) and sodium metabisulfite (0.500 mg/g) as antioxidants, glycerol (30.000 mg/g), ED TA (3.000 mg/g) as a chelating agent, and ethanol (30.000 mg/g).

### Example Formulation 65

Formulation 65 comprises phosphatidyl choline (44.198 mg/g) as a lipid, Tween 80 (50.802 mg/g) as a surfactant, phosphate (pH 6.5) buffer, benzyl alcohol or paraben (5.000 mg/g) as an antimicrobial, BHT (0,200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 66

Formulation 66 comprises phosphatidyl choline (46.453 mg/g) as a lipid, Tween 80 (51.047 mg/g) as a surfactant, phosphate (pH 6.5) buffer, benzyl alcohol or paraben (5.000 mg/g) as an antimicrobial. BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

Formulation 67 comprises phosphatidyl choline (51.221 mg/g) as a lipid, Tvveen 80 (43.779 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 68

Formulation 68 comprises phosphatidyl choline (54.167 mg/g) as a lipid, Tween 80 (43.333 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 69

Formulation 69 comprises phosphatidyl choline (66.440 mg/g) as a lipid, Brij 98 (23.560 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g). Example formulation 69 is an emulsion.

### Example Formulation 70

Formulation 70 comprises phosphatidyl choline (66.440 mg/g) as a lipid, Brij 98 (23.560 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g). Example formulation 70 is a suspension.

### Example Formulation 71

Formulation 71 comprises phosphatidyl choline (66.440 mg/g) as a lipid, Brij 98 (23.560 mg/g) as a surfactant, phosphate (pH 6.5) buffer, benzyl alcohol or paraben (5.000 mg/g) as an antimicrobial, BHT (0.200 mg/g) and sodium metabisulfite (0,500 mg/g) as antioxidants, glycerol (30.000 mg/g), EDTA (3.000 mg/g) as a chelating agent, and ethanol (30.000 mg/g).

Formulation 72 comprises phosphatidyl choline (40.000 mg/g) as a lipid, Tween 80 (50.000 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g). Example formulation 72 is an emulsion.

### Example Formulation 73

Formulation 73 comprises phosphatidyl choline (40.000 mg/g) as a lipid, Tween 80 (50.000 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g). Example formulation 73 is a suspension.

### Example Formulation 74

Formulation 74 comprises phosphatidyl choline (40.000 mg/g) as a lipid, Tween 80 (50.000 mg/g) as a surfactant, acetate (pH 5.5) buffer, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 75

Formulation 75 comprises phosphatidyl choline (40.000 mg/g) as a lipid, Tween 80 (50.000 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 76

Formulation 76 comprises phosphatidyl choline (40.000 mg/g) as a lipid, Brij 98 (50.000 mg/g) as a surfactant, phosphate (pH 6.5) buffer, benzalkonium chloride (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 77

Formulation 77 comprises phosphatidyl choline (40.000 mg/g) as a lipid, Tween 80 (50.000 mg/g) as a surfactant, phosphate (pH 6.5) buffer, benzyl alcohol or paraben (5,000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 78

Formulation 78 comprises phosphatidyl choline (66.440 mg/g) as a lipid, Brij 98 (23.560 mg/g) as a surfactant, phosphate (pH 6.5) buffer, benzalkonium chloride (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 79

Formulation 79 comprises phosphatidyl choline (66.440 mg/g) as a lipid, Brij 98 (23.560 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 80

Formulation 80 comprises phosphatidyl choline (40.000 mg/g) as a lipid, Tween 80 (50.000 mg/g) as a surfactant, acetate (pH 5.5) buffer, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 81

Formulation 81 comprises phosphatidyl choline (40.000 mg/g) as a lipid, Tween 80 (50.000 mg/g) as a surfactant, acetate (pH 5.5) buffer, benzyl alcohol or paraben (5.000 mg/g) as an antimicrobial. BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 82

Formulation 82 comprises phosphatidyl choline (44.444 mg/g) as a lipid, Tween 80 (55.556 mg/g) as a surfactant, acetate (pH 5.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 83

Formulation 83 comprises phosphatidyl choline (66.440 mg/g) as a lipid, Tween 80 (23.560 mg/g) as a surfactant, acetate (pH 5.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 84

Formulation 84 comprises phosphatidyl choline (54.000 mg/g) as a lipid, Tween 80 (36.000 mg/g) as a surfactant, acetate (pH 4) buffer, benzyl alcohol or paraben (5.000 mg/g) as an antimicrobial, BHA (0.200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 85

Formulation 85 comprises phosphatidyl choline (50.000 mg/g) as a lipid, Tween 80 (40.000 mg/g) as a surfactant, acetate (pH 4) buffer, benzyl alcohol or paraben (5.000 mg/g) as an antimicrobial, BHA (0.200 mg/g) and sodium metabisulfite (0.500 mg/g) as antioxidants, glycerol (30.000 mg/g), EDTA (3.000 mg/g) as a chelating agent, and ethanol (30.000 mg/g)

### Example Formulation 86

Formulation K6 comprises phosphatidyl choline (48.61 1 mg/g) as a lipid. Tween 80 (38.889 mg/g) as a surfactant, acetate (pH 4) buffer, benzyl alcohol or paraben (5.000 mg/g) as an antimicrobial, BH[Lambda] (0.200 mg/g) and sodium metabisulfite (0,500 mg/g) as antioxidants, glycerol (30.000 mg/g), EDTA (3.000 mg/g) as a chelating agent, and ethanol (30.000 mg/g).

### Example Formulation 87

Formulation 87 comprises phosphatidyl choline (46.575 mg/g) as a lipid, Tween 80 (38,425 mg/g) as a surfactant, acetate (pH 4) buffer, benzyl alcohol or paraben (5.000 mg/g) as an antimicrobial, BHA (0.200 mg/g) and sodium metabisulf[iota]te (0.500 mg/g) as antioxidants, glycerol (30,000 mg/g), EDTA (3.000 mg/g) as a chelating agent, and ethanol (30.000 mg/g). Example formulation 87 is an emulsion.

### Example Formulation 88

Formulation 88 comprises phosphatidyl choline (46.575 mg/g) as a lipid, Tween 80 (38.425 mg/g) as a surfactant, acetate (pH 4) buffer, benzyl alcohol or paraben (5.000 mg/g) as an antimicrobial, BHA (0.200 mg/g) and sodium metabisulf[iota]te (0.500 mg/g) as antioxidants, glycerol (30.000 mg/g), EDTA (3.000 mg/g) as a chelating agent, and ethanol (30.000 mg/g). Eample formulation 88 is suspension.

### Example Formulation 89

Formulation 89 comprises phosphatidyl choline (46.575 mg/g) as a lipid, Tween 80 (38.425 mg/g) as a surfactant, acetate (pH 4) buffer, benzyl alcohol or paraben (5.000 mg/g) as an antimicrobial, BUT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 90

Formulation 90 comprises phosphatidyl choline (50.000 mg/g) as a lipid, Tween 80 (40.000 mg/g) as a surfactant, acetate (pH 4.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 91

Formulation 91 comprises phosphatidyl choline (94.444 mg/g) as a lipid, Tween 80 (75.556 mg/g) as a surfactant, acetate (pH 4) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 92

Formulation 92 comprises phosphatidyl choline (46.712 mg/g) as a lipid, Tween 80 (38.288 mg/g) as a surfactant, acetate (pH 4) buffer, benzyl alcohol or paraben (5.000 mg/g) as an antimicrobial. BHT (0.200 mg/g) and sodium metabisulfite (0.500 mg/g) as antioxidants, glycerol (30.000 mg/g). EDTA (3.000 mg/g) as a chelating agent, and ethanol (30.000 mg/g).

### Example Formulation 93

Formulation 93 comprises phosphatidyl choline (48.889 mg/g) as a lipid, Tween 80 (39.111 mg/g) as a surfactant, acetate (pH 4) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 94

Formulation 94 comprises phosphatidyl choline (39.721 mg/g) as a lipid, Tween 80 (50.279 mg/g) as a surfactant, phosphate (pH 6.5) buffer, benzyl alcohol or paraben (5.25 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 95

Formulation 95 comprises phosphatidyl choline (90.000 mg/g) as a lipid, phosphate buffer (pH 6.5), benzyl alcohol or paraben as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 96

Formulation 96 comprises phosphatidyl choline (68.700 mg/g) as a lipid, Tween 80 (8.500 mg/g) as a surfactant, phosphate (pH 7.5) buffer, BHT (0.200 mg/g) and sodium metabisulfite (0.500 mg/g) as antioxidants, benzyl alcohol or paraben (5.000 mg/g) as an antimicrobial, glycerol (30.000 mg/g), EDTA (1.000 mg/g) as a chelating agent, and ethanol (36.51 mg/g).

### Example Formulation 97

Formulation 97 comprises phosphatidyl choline (71.460 mg/g) as a lipid, Tween 80 (4.720 mg/g) as a surfactant, phosphate (pH 7.5) buffer. BHA (0.200 mg/g) and sodium metabisulfite (0.500 mg/g) as antioxidants, benzyl alcohol or paraben (5.000 mg/g) as an antimicrobial, glycerol (50.000 mg/g), EDTA (3.000 mg/g) as a chelating agent and ethanol (35.000 mg/g).

### Example Formulation 98

Formulation 98 comprises phosphatidyl choline (71.460 mg/g) as a lipid, Tween 80 (4.720 mg/g) as a surfactant, phosphate (pH 7.8) buffer. BHA (0.200 mg/g) and sodium metabisulfite (0.500 mg/g) as antioxidants, benzyl alcohol or paraben (5.000 mg/g) as an antimicrobial, glycerol (15.000 mg/g), EDTA (3.000 mg/g) as a chelating agent, and ethanol (35.000 mg/g).

### Example Formulation 99

Formulation 99 comprises phosphatidyl choline (71.460 mg/g) as a lipid, Tween 80 (4.720 mg/g) as a surfactant, phosphate (pH 7.8) buffer, BHA (0.200 mg/g) and sodium metabisulfite (0.500 mg/g) as antioxidants, glycerol (50.000 mg/g), EDTA (3.000 mg/g) as a chelating agent, and ethanol (15.000 mg/g).

### Example Formulation 100

Formulation 100 comprises phosphatidyl choline (71.4600 mg/g) as a lipid, Tween 80 (4.720 mg/g) as a surfactant, phosphate (pH 7.5) buffer, BHA (0.200 mg/g) and sodium metabisulfite (0.500 mg/g) as antioxidants, glycerol (50.000 mg/g), EDTA (3.000 mg/g) as a chelating agent, and ethanol (35.000 mg/g).

### Example Formulation 101

Formulation 101 comprises phosphatidyl choline (46.575 mg/g) as a lipid, Tween 80 (38.425 mg/g) as a surfactant, phosphate (pi I 4) buffer, BHT (0.500 mg/g) and sodium metabisulfite (0.200mg/g) as antioxidants, and EDTA (3.000 mg/g) as a chelating agent. Example formulation 101 is an emulsion.

### Example Formulation 102

Formulation 102 comprises phosphatidyl choline (46.575 mg/g) as a lipid, Tween 80 (38.425 mg/g) as a surfactant, phosphate (pH 4) buffer, BHT (0.500 mg/g) and sodium metabisulfite (0.200mg/g) as antioxidants, and EDTA (3.000 mg/g). Example formulation 102 is a suspension.

### Example Formulation 103

Formulation 103 comprises phosphatidyl choline (54.643 mg/g) as a lipid, Tween 80 (30.357 mg/g) as a surfactant, phosphate (pH 4) buffer, BHA (0.500 mg/g) and sodium metabisulfite (0.200mg/g) as antioxidants, and EDTA (3.000 mg/g) as a chelating agent.

### Example Formulation 104

Formulation 104 comprises phosphatidyl choline (39.72 mg/g)as a lipid, Tween 80 (50.279 mg/g) as surfactant, phosphate (pH 6.5) buffer, benzyl alcohol or paraben (5.00 mg/g) as an antimicrobial, BHT (0.200 mg/g) and sodium metabisulfite (0.500 mg/g) as antioxidants, glycerol (30.000 mg/g) as emollient, EDTA (3.000 mg/g) as the chelating agent, and ethanol (30.000 mg/g).

### Example Formulation 105

Formulation 105 comprises phosphatidyl choline (90.00 mg/g) as a lipid, phosphate (pH 6.5) buffer, benzyl alcohol or paraben as antimicrobial (5.000 mg/s), BHT (0.200 mg/g) and sodium metabisulfite (0.500 mg/g) as antioxidants, glycerol (30.000 mg/g) as emollient, EDTA (3.000 mg/g) as the chelating agent, and ethanol (30.000 mg/g).

### Example Formulation 106

Formulation 106 comprises phosphatidyl choline (46.57 mg/g) as a lipid, Tween 80 (38.425 mg/g) as a surfactant, phosphate (pH 4) buffer, BHT (0.500 mg/g) and sodium metabisulfite (0.200mg/g) as antioxidants, and EDTA (3.000 mg/g) as the chelating agent. Formulation 106 is formulated as an emulsion.

### Example Formulation 107

Formulation 107 comprises phosphatidyl choline (46.57 mg/g) as a lipid, Tween 80 (38.425 mg/g) as a surfactant, phosphate (pH 4) buffer, BHT (0.500 mg/g) and sodium metabisulfite (0.200mg/g) as antioxidants, and EDTA (3.000 mg/g) as the chelating agent. Formulation 107 as a suspension.

### Example Formulation 108

Formulation 108 comprises phosphatidyl choline (54.64 mg/g)as a lipid, Tween 80 (30.357 mg/g) as a surfactant, phosphate (pH 4) buffer, BHA (0.500 mg/g) and sodium metabisulfite (0.200mg/g) as antioxidants, EDTA (3.000 mg/g) as the chelating agent.

### Example Formulation 109

Formulation 109 comprises phosphatidyl glycerol and lysophospholipid (46.364 mg/g) as a lipid, Brij 98 (38.636 mg/g) as a surfactant, acetate (pH 4) buffer, benzyl alcohol or paraben (5.000 mg/g) as an antimicrobial, BHT (0.200 mg/g) as an antioxidant, glycerol (30.000 mg/g), EDTA (3.000 mg/g) as a chelating agent, and ethanol (25.000 mg/g).

### Example Formulation 110

Formulation 110 comprises phosphatidyl glycerol and lysophospholipid (46.364 mg/g) as a lipid, Brij 98 (38.636 mg/g) as a surfactant, acetate (pH 4) buffer, benzyl alcohol or paraben (5.000 mg/g) as an antimicrobial, BHT (0.200 mg/g) as an antioxidant, EDTA (3.000 mg/g) as a chelating agent, and ethanol (20.000 mg/g).

### Example Formulation 111

Formulation 111 comprises phosphatidyl glycerol and lysophospholipid (46.098 mg/g) as a lipid, Brij 98 (43.902 mg/g) as a surfactant, acetate (pH 5) buffer, benzyl alcohol or paraben (5.000 mg/g) as an antimicrobial, BHT (0.200 mg/g) as an antioxidant, glycerol (15.000 mg/g), EDTA (3.000 mg/g) as a chelating agent, and ethanol (30.000 mg/g).

### Example Formulation 112

Formulation 1 12 comprises phosphatidyl glycerol and lysophospholipid (43.537 mg/g) as a lipid, Brij 98 (41.463 mg/g) as a surfactant, acetate (pH 5) buffer, benzyl alcohol or paraben (5.000 mg/g) as an antimicrobial, BHT (0.200 mg/g) as an antioxidant, glycerol (30.000 mg/g), and EDTA (3.000 mg/g) as a chelating agent.

### Example Formulation 113

Formulation 113 comprises phosphatidyl glycerol and lysophospholipid (45.000 mg/g) as a lipid, Brij 98 (45.000 mg/g) as a surfactant, acetate (pH 5) buffer, benzyl alcohol or paraben (5.000 mg/g) as an antimicrobial, BHT (0.200 mg/g) as an antioxidant, EDTA (3.000 mg/g) as a chelating agent, and ethanol (30,000 mg/g).

### Example Formulation 114

Formulation 114 comprises phosphatidyl glycerol and lysophospholipid (59.492 mg/g) as a lipid, Brij 98 (30.508 mg/g) as a surfactant, acetate (pH 6.5) buffer, benzyl alcohol or paraben (5.000 mg/g) as an antimicrobial, BHT (0.200 mg/g) as an antioxidant, glycerol (30.000 mg/g), EDTA (3.000 mg/g) as a chelating agent, and ethanol (30.000 mg/g).

### Example Formulation 115

Formulation 1 15 comprises phosphatidyl glycerol and lysophospholipid (39.054 mg/g) as a lipid, Brij 98 (45,946 mg/g) as a surfactant, acetate (pH 6.5) buffer, benzyl alcohol or paraben (5.000 mg/g) as an antimicrobial, BHT (0.200 mg/g) as an antioxidant, and EDTA (3.000 mg/g) as a chelating agent.

### Example Formulation 116

Formulation 116 comprises phosphatidyl glycerol and lysophospholipid (35.854 mg/g) as a lipid, Brij 98 (34.146 mg/g) as a surfactant, acetate (pH 6.5) buffer, benzyl alcohol or paraben (5.000 mg/g) as an antimicrobial, BHT (0.200 mg/g) as an antioxidant, glycerol (30.000 mg/g), and EDTA (3.000 mg/g) as a chelating agent.

### Example Formulation 117

Formulation 117 comprises phosphatidyl choline and lysophospholipid (50.000 mg/g) as a lipid, Tween 80 (40.000 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 118

Formulation 118 comprises phosphatidyl choline and lysophospholipid (38.571 mg/g) as a lipid, Tween 80 (51.429 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g), and ethanol (30.000 mg/g).

### Example Formulation 119

Formulation 119 comprises phosphatidyl choline and lysophospholipid (41.954 mg/g) as phospholipid, Tween 80 (50.546 mg/g) as surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g), and ethanol (30.000 mg/g).

### Example Formulation 120

Formulation 120 comprises phosphatidyl choline and lysophospholipid (42.632 mg/g) as a lipid, Tween 80 (47.368 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 121

Formulation 121 comprises phosphatidyl choline and lysophospholipid (46.098 mg/g) as a lipid, Tween 80 (43.902 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 122

Formulation 122 comprises phosphatidyl choline and lysophospholipid (39.721 mg/g) as a lipid, Twecn 80 (50.279 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 123

Formulation 123 comprises phosphatidyl choline and lysophospholipid (44.198 mg/g) as a lipid, Tween 80 (50.802 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 124

Formulation 124 comprises phosphatidyl choline and lysophospholipid (46.453 mg/g) as a lipid, Tween 80 (51.047 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 125

Formulation 125 comprises phosphatidyl choline and lysophospholipid (51.221 mg/g) as a lipid. Tween 80 (43.779 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 126

Formulation 126 comprises phosphatidyl choline (54.167 mg/g) as a lipid, Tween 80 (43.333 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

### Example Formulation 127

Formulation 127 comprises phosphatidyl choline and lysophospholipid (66.440 mg/g) as a lipid, Brij 98 (23.560 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g) Example formulation 69 is an emulsion.

### Example Formulation 128

Formulation 128 comprises phosphatidyl choline and lysophospholipid (66.440 mg/g) as a lipid, Brij 98 (23.560 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g). Example formulation 70 is a suspension.

### Example Formulation 129

Formulation 129 comprises phosphatidyl choline and lysophospholipid (66.440 mg/g) as a lipid. Brij 98 (23.560 mg/g) as a surfactant, phosphate (pH 6.5) buffer, benzyl alcohol or paraben (5.000 mg/g) as an antimicr[upsilon]bial. BHT (0 200 mg/g) and sodium metabisulfite (0.500 mg/g) as antioxidants, glycerol (30.000 mg/g), EDTA (3.000 mg/g) as a chelating agent, and ethanol (30.000 mg/g).

It will be understood that the exact amounts of the components of the formula may be adjusted slightly without departing from the scope of the invention. For example, in each of the above formulations, the amount antimicrobial be anywhere from about 1 mg/g to about 15 mg/g, or about 5 m/g to about 12 mg/g, or 5.25 mg/g, 6, mg/6, 7 mg/g, 8 mg/g, 9 mg/g, 10 mg/g, or 10.25 mg/g. Furthermore, the antimicrobial can be a combination of ingredients, for example benzyl alcohol and parabenes (e.g., ethyl and/or propyl).

Example Formulations 1 through 129 may also optionally include thickeners such as pectin, xanthan gum. HPMC gel, methylcellulose or carbopol.

### Example 2

This experiment was conducted on 6 week old CD^{®} Hairless female rats (Charles River labs).

The DiO-labeled deformable vesicles were applied to the knee joints of four hairless rats at a dose of 10mg per joint. The test article (10mg per knee) was applied 2-5 times per day at 4 hour intervals for 3 days. The formulation , consisting of Soy Phosphatidylcholine (68.7 mg/g,) Tween 80 (8.5 mg/g) to form the vesicular component which contained the fluorescent species DIO + carbopol (12.5 mg/g) +preservatives, antioxidants and stabilisers in water (816 mg/g) was applied on to the skin of the joint and allowed to dry. Extra care was taken to avoid any mechanical damage to the skin. The animals were sacrificed following the last application of the test article. One knee of each animal was dissected and flash frozen in Acrytol Mounting Media (Leica Inc.) which contains 10% PolyVinyl Alcohol (PVA). The frozen 10µm thick sections was imaged and analyzed for DIO staining and number of synovial vesicle density as described below.

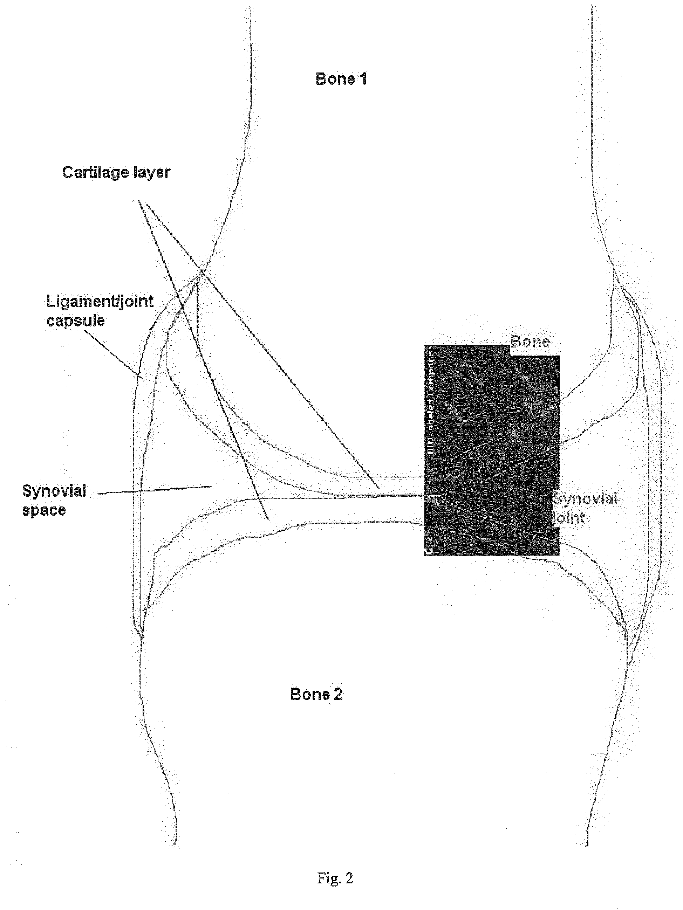

All collected joint tissues were cryo-sectioned and fixed (and stained with DAPI for cell location purposes). The images were recorded and show the localisation of the vesicles for use according to the formulation of the invention in Figures 1 and 2.

### Examples 3 to 5

Six clinical trials were conducted with Formulation X on patients with osteoarthritis of the knee. One efficacy measure was the change from baseline to the end of the study in patients' perception of joint stiffness and physical function. In studies CL-033-III-03 and CL-033-III-06, joint stiffness was assessed using two single items describing the severity of stiffness, both of which were measured using an 11-point numerical rating (NRS) on the stiffness subscale of the Western Ontario and McMaster Universities (WOMAC) Osteoarthritis Index (version 3.1) instrument. A measure of physical function was performed in all six studies using the WOMAC Osteoarthritis Index (version 3.1), which consisted of 17 single items describing the difficulty in performing daily activities. Each item was rated on a VAS or an 11-point numerical scale.

Studies CL-033-III-02, CL-033-III-03 and CL-033-III-06 examined the efficacy and safety of epicutaneously applied Formulation X (4.4 g or 2.2 g twice daily) or ketoprofen in Transfersome^{®} gel administered for 12 weeks. CL-033-III-03 was an active-controlled study comparing 12 weeks of Formulation X (4.4 g or 2.2 g twice daily) with celecoxib (100 mg twice daily), oral placebo or ketoprofen in Transfersome^{®} gel.

Studies CL-033-II-03, CL-033-III-04 and CL-033-III-05 provided additional supportive efficacy and safety data for Formulation X combination regimens. Study CL-033-II-03 evaluated 6 weeks' treatment with Formulation X (4.8 g twice daily) in combination with celecoxib (100 mg twice daily) or oral placebo versus ketoprofen in Transfersome^{®} gel. Study CL-033-III-04 compared 12 weeks' treatment with Formulation X (4.95 g, 2.65 g and 1.45 g twice daily) in combination with oral naproxen (500 mg twice daily) or oral placebo versus ketoprofen in Transfersome^{®} gel. CL-033-III-05 was a double-blind extension of study CL-033-III-04 over 52 weeks in which patients received Formulation X 4.95 g in combination with oral naproxen (500 mg twice daily) versus ketoprofen in Transfersome^{®} gel plus oral placebo.

Efficacy outcomes reported here focus on the 943 patients who received Formulation X alone.

"Formulation X" is as set out below, and is in accordance with example formulation 96 as described above:

| Trade name | Concentration [mg/g] |
|---|---|
| Soy Phosphatidylcholine | 68.70 |
| Polysorbate 80 | 8.50 |
| Butylated hydroxytoluene | 0.20 |
| (BHT) | |
| Disodium edetate | 1.00 |
| Methyl paraben | 2.50 |
| Ethyl paraben | 2.50 |
| Glycerol | 30.00 |
| Sodium hydroxide | 6.30 |
| Disodium hydrogenphosphate | 7.55 |
| dodecahydrate | |
| Sodium dihydrogenphosphate | 0.61 |
| dihydrate | |
| Carbopol | 12.50 |
| Linalool | 1.00 |
| Ethanol (96%) | 36.51 |
| Benzyl alcohol | 5.25 |
| Purified water | 816.38 |
| Sodium metabisulphate | 0.50 |

### Example 3

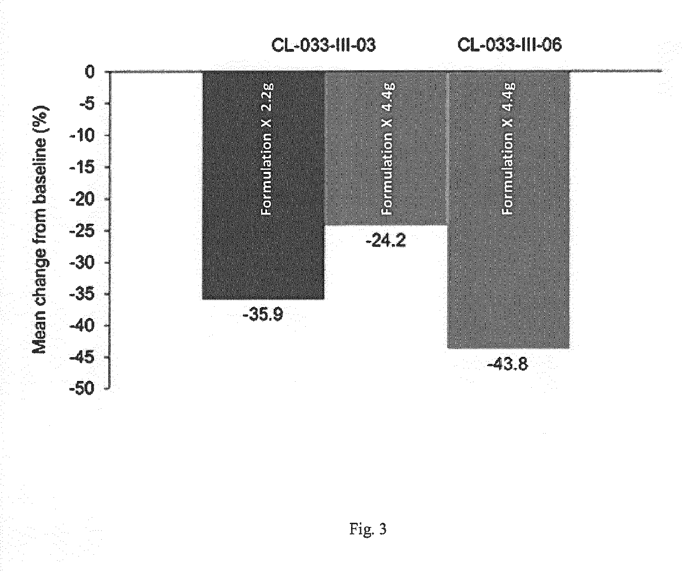

In studies CL-033-III-03 and CL-033-III-06, the difference in joint stiffness from baseline to the end of the 3-month treatment period with Formulation X alone was measured. Joint stiffness ratings improved by up to 43.8% in 753 patients (Figure 3) (CSR CL-033-III-03; CSR CL-033-III-06).

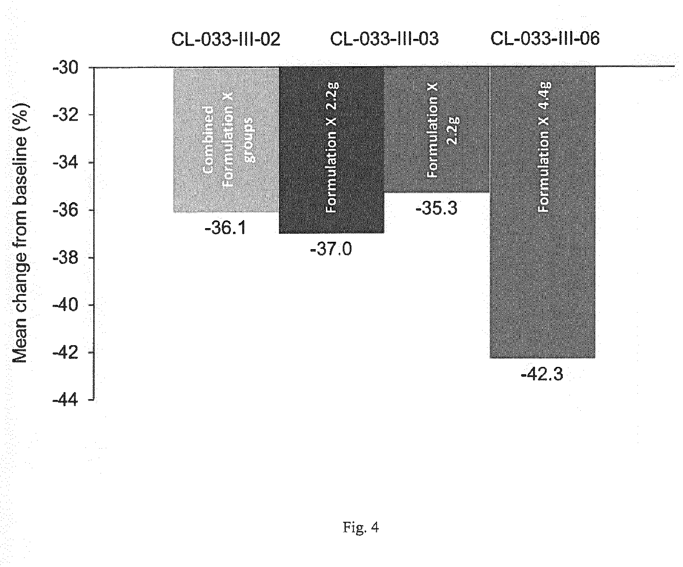

At the end of the 3-month treatment period in the clinical trials assessing Formulation X alone, enhanced physical function of up to 42.3% was noted in 943 patients (Figure 4) (CSR CL-033-III-02; Conaghan et al. 2012; Rother et al. 2012a). Quality of life, assessed using the EURO QoL, improved by almost 40% in patients who received Formulation X in study CL-033-III-02.

Improved physical function was reported with Formulation X alone and in combination with celecoxib at the end of the 6-week treatment period in study CL-033-II-03 (CSR CL-033-II-03): 10.2% improvement for Formulation X alone versus 16.6% for Formulation X/celecoxib (p=0.01) and 14.6% for topical ketoprofen in Transfersome^{®} gel (p=0.077).

In study CL-033-III-04, both the Formulation X/naproxen arm and the Formulation X/placebo arm showed improvements in physical function after 3 months (41.8% and 29.9%, respectively) (CSR CL-033-III-04), and this was maintained after 52 weeks of treatment in study CL-033-III-05 (42.3% vs 8.6% for topical ketoprofen in Transfersome^{®} gel [p=0.05]) (CSR CL-033-III-05). An improvement of 36.5% in mental health scores (SF-36 health survey) was also observed for both Formulation X-containing study arms in study CL-033-III-04.

### Example 4

Pain, joint mobility and physical function outcomes were compared in study CL-033-III-03. Celecoxib (Celebrex^{®}) is known to reduce pain in OA and was considered to be an appropriate active comparator in this study, controlling for bias and providing a reference for comparing the treatment effect of Formulation X. In study CL-033-III-03 patients were assigned to treatment with celecoxib (100 mg twice daily), placebo or active comparator (diclofenac) for 6 weeks (McKenna et al. 2001).

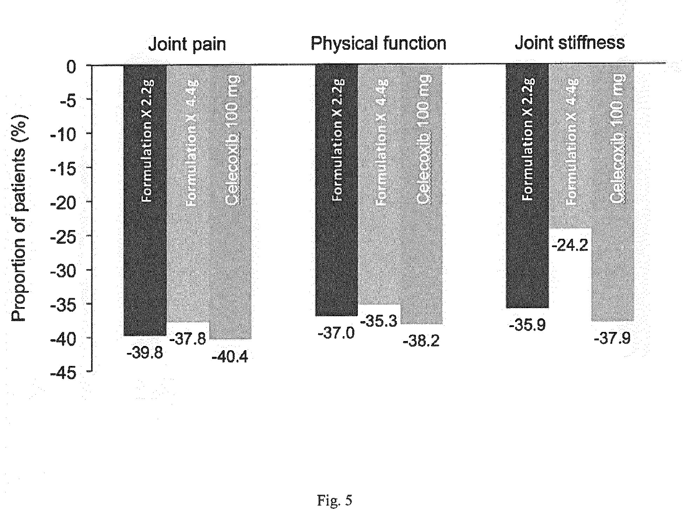

At the end of 3 month treatment period in study CL-033-III-03, Formulation X was associated with reductions in joint pain (up to 39.8%) and stiffness (up to 35.9%), and an improvement in physical function (up to 37.0%) that were comparable with the effects of oral celecoxib (40.4%, 37.9% and 38.2%, respectively) (Figure 5) (CSR CL-033-III-03; Conaghan et al. 2012). Reductions in pain achieved with Formulation X were statistically significantly non-inferior to oral celcoxib, and superior to oral placebo at a magnitude similar to that of celecoxib (Table 1). These response rates for Formulation X compare favourably with those reported for patients who received therapy with oral celecoxib for 12 weeks (percentage reduction in WOMAC pain, function and stiffness approximately 30%, 26% and 26%, respectively [estimated data]) in a large double-blind, placebo-controlled study by Bensen et al. (1999). This demonstrates consistency between the efficacy data for Formulation X in Study CL-033-III-03 and published celecoxib data.

**Table 3. Confirmatory analysis of effect size (Mann-Whitney estimator) for WOMAC pain and function after 3 months of therapy with Formulation X in Study CL-033-III-03. The pre-specified benchmark for superiority was MW >0.5. Noninferiority versus celecoxib was tested for pain and function in exploratory and post hoc analyses using a pre-specified lower equivalence margin of MW = 0.4.**

| | 2.2 g Formulation X (n=238) | 4.4 g Formulation X (n=234) | Oral cel ecoxi b 100 mg b.i.d. (n=233) | Oral placebo matching celecoxib (n=227) |
|---|---|---|---|---|
| **WOMAC pain subscale score** | | | | |
| Effect size versus oral placebo | MW=0.6006 | MW=0.5779 | MW=0.5873 | - |
| | LB: 0.5404 | LB: 0.5176 | LB: 0.5268 | |
| | ^{∗}p=0.0001 | ^{∗}p=0.0019 | ^{∗}p=0.0006 | |
| Effect size versus oral cel ecoxi b | MW=0.5101 | MW=0.4897 | - | - |
| | LB: 0.4506 | LB: 0.4299 | | |
| | ^{∗∗}p=0.3526 | ^{∗∗}p=0.6504 | | |

| **WOMAC function subscale score** | | | | |
|---|---|---|---|---|
| Effect size versus oral placebo | MW=0.6054 | MW=0.5788 | MW=0.5949 | - |
| | LB: 0.5452 | LB: 0.5184 | LB: 0.5344 | |
| | ^{∗}p<0.0001 | ^{∗}p=0.0017 | ^{∗}p=0.0002 | |
| Effect size versus oral cel ecoxi b | MW=0.5062 | MW=0.4829 | - | - |
| | LB: 0.4467 | LB: 0.4232 | | |
| | ^{∗∗}p=0.4076 | ^{∗∗}p=0.7388 | | |

| | | | | |
|---|---|---|---|---|
| LB, lower boundary of confidence interval; MW, Mann-Whitney estimator; SD, standard deviation; WOMAC, Western Ontario and McMaster Universities. ^{∗}P-values calculated using Wilcoxon-Mann-Whitney U test based on one-sided 98.75% confidence intervals. ^{∗∗}P-values represent test for superiority. | | | | |

### Example 5

The results of a meta-analysis of the change from baseline in WOMAC Osteoarthritis Index pain and function subscales from five of the clinical studies (CL-033-II-03; CL-033-111-02; CL-033-III-03; CL-033-III-04; CL-033-III-06) suggest that the treatment effects seen with Formulation X in the clinical trials represent genuine improvements in outcomes for patients with OA, rather than being only due to a placebo response which has been reported in interventional trials in OA (Zhang et al. 2010).

The change from baseline in WOMAC Osteoarthritis Index pain and function from each study were standardised to a 0-100 scale. The resulting pre-post effect size (ES) was calculated as the standardised difference of the change from baseline of the WOMAC pain subscale score at various times. The results were compared with data from the study by Zhang et al. (2010) who examined determinants of the placebo response in a meta-analysis of 198 randomised OA trials.

**Table 4. Effect size for individual studies and their meta-analysis evaluated by the standardised difference and 95 % CIs applied for WOMAC pain and function after 6 weeks of treatment with Formulation X (ITT analysis, LOCF)**

| | | **Standard difference** | **95% CI** | **N1/N2** |
|---|---|---|---|---|
| **Pain** | | | | |
| Combined Formulation X groups | | | | |
| | CL-033-II-03 | 0.6 | 0.42-0.77 | 126/126 |
| | CL-033-III-02 | 1.28 | 1.13-1.42 | 190/190 |
| | CL-033-III-03 | 1.03 | 0.94-1.13 | 472/472 |
| | CL-033-III-04 | 1.08 | 0.92-1.23 | 162/162 |
| | CL-033-III-06 | 1.06 | 0.94-1.18 | 281/281 |
| Combined Formulation X studies (Hedges-Olkin) | | 1.04 | 0.98-1.09 | 1231/1231 |
| Meta-analysis (Zhang et al.) | | | | |
| | All placebo | 0.54 | 0.49-0.6 | |
| | Topical placebo | 0.63 | 0.47-0.8 | |

| Function Combined Formulation X groups | | | | |
|---|---|---|---|---|
| | CL-033-II-03 | 0.64 | 0.46-0.82 | 127/127 |
| | CL-033-III-02 | 0.88 | 0.73-1.02 | 185/185 |
| | CL-033-III-03 | 0.97 | 0.87-1.05 | 472/472 |
| | CL-033-III-04 | 0.95 | 0.80-1.11 | 162/162 |
| | CL-033-III-06 | 1.01 | 0.89--1.13 | 281/281 |
| Combined Formulation X studies (Hedges-Olkin) | | 0.93 | 0.87-0.93 | 1061/1061 |
| Meta-analysis (Zhang et al.) | | | | |
| | All placebo | 0.49 | 0.44-0.54 | |

Higher ESs for pain relief for Formulation X studies versus the data of Zhang et al. (2010) were reported in studies using a flare design (ES: 1.00 [95% CI: 0.93-1.07]), and in patients with high (ES: 1.08 [95% CI: 1.00-1.17]) or low baseline pain severity (ES: 1.03 [95% CI: 0.95-1.11]). These data, and the magnitude of the ES with Formulation X, demonstrate that its effect is unlikely to be solely as a result of a placebo response.

### Example 6

A survey was conducted among a community for older people to assess the effect of Formulation X when used by arthritis sufferers in a daily routine setting, and to understand their views on the benefits and attributes of the formulation.

In total, 390 subjects with OA were recruited to test Formulation X on an index joint. The baseline characteristics indicated that this was a fairly standard population of individuals with OA. The average age of respondents was 65 years, 85% had comorbid conditions and 83% reported having OA in more than one joint. From the enrolled population of 390 subjects, a population of 177 subjects have completed 3 weeks of treatment with Formulation X. Among this group, the average baseline pain was 6.85 and the average baseline stiffness was 5.29, both measured using a 10-point Visual Analogue Scale (VAS).

From the enrolled population of 390 subjects, 334 used Formulation X at least once, and after 1, 2 and 3 weeks, 333, 248 and 177 subjects were still using Formulation X, respectively. A variety of reasons were given for stopping treatment, including side effects (discussed later) and finishing the two tubes of Formulation X gel.

Although subjects were requested to use the gel on one index joint only, 172 (51.5%) reported using the gel on one or more joints. The joints that were treated are listed in Table 5.

**Table 5: Joints treated with Formulation X**

| **Knee, n (%)** | **Elbow, n (%)** | **Wrist, n (%)** | **Shoulder, n (%)** | **Ankle, n (%)** | **Finger, n (%)** | **Other, n (%)** |
|---|---|---|---|---|---|---|
| 222 (66.5) | 21 (6.3) | 57 (17.1) | 59 (17.7) | 35 (10.5) | 91 (27.2) | 87 (26.0) |

Among the total population, the average baseline pain was 6.92 and the average baseline stiffness was 5.15. These values were similar to the baseline values of the subjects who completed 3 weeks' treatment (6.85 for pain and 5.29 for stiffness), which indicated that the 3-week completers were representative of the entire dataset and provided the opportunity to evaluate the effect of Formulation X on pain and stiffness in a subset of subjects who had received 3 weeks' treatment.

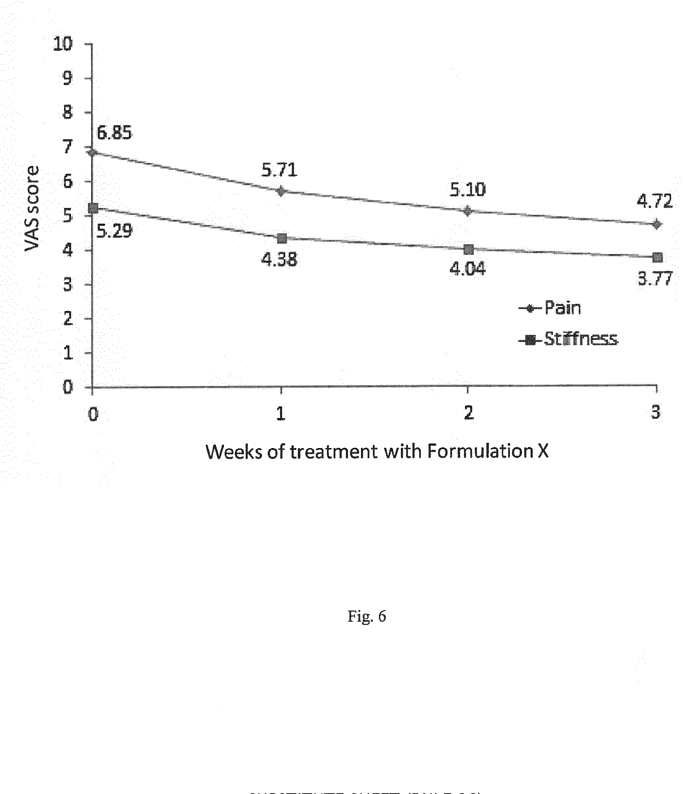

Three weeks of treatment saw a decrease of the average pain score by 2.13 (31.1%) and an improvement in the average stiffness score of 1.52 (28.7%) (Figure 6).

### Responder analysis

After 3 weeks of treatment with Formulation X, 73.4% and 57.1% of subjects reported a ≥1 point or ≥2 point improvement in pain in their index j oint, respectively, while 64.8% and 46.1% of subjects reported a ≥1 point or ≥2 point improvement in stiffness in their index joint, respectively.

Although very few patients reported when they initially started to see a beneficial effect, over half (52.8%) reported an improvement of ≥1 point after one week of treatment.

Analysis of the response according to the level of starting pain indicated that the most pronounced improvements in pain were observed in the subjects with moderate or higher pain at baseline (Table 6).

**Table 6: Analysis of effect according to baseline pain**

| **Starting pain** | **Average starting pain score** | **No subjects** | **Av delta at 3 weeks** | **Av delta as % of starting score** |
|---|---|---|---|---|
| 1-4 | 3.64 | 28 | 0.39 | 10.7 |
| 5-7 | 5.89 | 65 | 1.89 | 32.1 |
| 8-10 | 8.59 | 84 | 2.89 | 33.6 |

In an analysis of feedback on the formulation, 97% of respondents reported that Formulation X was equal or better than other products with respect to its effects on pain, while 74% reported that it was slightly or much better (Table 7). Similarly, with respect to stiffness, 99% reported that Formulation X was equal or better than other OA products, while 67% reported that it was slightly or much better.

Overall, 94% of respondents reported that Formulation X was equally or better tolerated than other products while over half of respondents reported that it was slightly or much better. A similar pattern was observed when respondents were questioned about ease of use with Formulation X compared with other products, with only 8% reporting that the formulation was worse than other products.

**Table 7: Respondent views on Formulation X compared with other OA products**

| | **Pain (131/177), n (%)** | **Stiffness (124/177), n (%)** | **Side effects (108/177), n (%)** | **Ease of use (125/177), n (%)** |
|---|---|---|---|---|
| **Worse** | 4 (3) | 1 (1) | 6 (6) | 10 (8) |
| **Equal** | 31 (24) | 40 (32) | 44 (41) | 53 (42) |
| **Slightly better** | 52 (40) | 45 (36) | 17 (16) | 26 (21) |
| **Much better** | 44 (34) | 38 (31) | 41 (38) | 36 (29) |

The positive findings in this observational study reinforce those of the clinical trials with Formulation X (Examples 3 to 5) that showed that this innovative, drug-free treatment is efficacious and well tolerated for the treatment of pain and stiffness associated with OA. The benefits of Formulation X are further reflected in the high patient satisfaction reported by those using the product. As Formulation X improves symptoms of OA without active pharmaceutical ingredients, it represents a real breakthrough for the management of this condition, allowing it to be used with confidence in a population where comorbid conditions and concomitant medication use is widespread.

## Claims

1. A vesicular formulation consisting essentially of one or more phospholipids, one or more non-ionic surfactants and a pharmaceutically acceptable carrier, and optionally further containing one or more of the following ingredients: co-solvents, chelators, buffers, antioxidants, preservatives, microbicides, emollients, humectants, lubricants and thickeners, for use in the treatment of reduced mobility associated with a loss of lubrication and/or structural integrity and/or swelling of a collagen structure in an animal by topically applying the vesicular formulation to the skin surrounding the collagen structure.

2. The vesicular formulation for use according to claim 1, wherein the collagen structure is cartilage within an articulated joint.

3. The vesicular formulation for use according to claim 1, wherein the collagen structure is a tendon or a ligament.

4. The vesicular formulation for use according to claim 2, wherein the loss of lubrication and/or structural integrity and/or swelling is due to degradation of phospholipids upon the surface of cartilage within an articulated joint.

5. The vesicular formulation for use according to claim 3, wherein the loss of lubrication and/or structural integrity and/or swelling is due to depletion of phospholipids between the tendon or ligament and a surface over which it moves.

6. The vesicular formulation for use according to any one of claims 1 to 5, wherein the reduced mobility is treated by visco-supplementation.

7. The vesicular formulation for use according to claim 3, for the treatment of tendonitis.

8. The vesicular formulation for use according to claim 3, for the treatment of carpal tunnel syndrome.

9. The vesicular formulation for use according to any one of claims 1 to 8, wherein the animal is a human, a companion animal or an agricultural animal.

10. The vesicular formulation for use according to any one of claims 1 to 9, wherein the animal is a human and is from about 45 to about 85 years old.

## Patentansprüche

1. Vesikuläre Formulierung, die im Wesentlichen aus einem oder mehreren Phospholipiden, einem oder mehreren nichtionischen Tensiden und einem pharmazeutisch annehmbaren Träger besteht und optional ferner einen oder mehrere der folgenden Bestandteile enthält: Co-Lösungsmittel, Chelatbildner, Puffer, Antioxidantien, Konservierungsmittel, Mikrobizide, Erweichungsmittel, Feuchthaltemittel, Schmiermittel und Verdickungsmittel, zur Verwendung in der Behandlung von verminderter Beweglichkeit, die mit einem Verlust an Schmierung und/oder struktureller Integrität und/oder einer Schwellung einer Kollagenstruktur assoziiert ist, in einem Tier durch topisches Auftragen der vesikulären Formulierung auf die die Kollagenstruktur umgebende Haut.

2. Vesikuläre Formulierung zur Verwendung nach Anspruch 1, wobei es sich bei der Kollagenstruktur um Knorpel innerhalb eines artikulierten Gelenks handelt.

3. Vesikuläre Formulierung zur Verwendung nach Anspruch 1, wobei es sich bei der Kollagenstruktur um eine Sehne oder ein Band handelt.

4. Vesikuläre Formulierung zur Verwendung nach Anspruch 2, wobei der Verlust an Schmierung und/oder struktureller Integrität und/oder die Schwellung auf den Abbau von Phospholipiden auf der Knorpeloberfläche innerhalb eines artikulierten Gelenks zurückzuführen ist.

5. Vesikuläre Formulierung zur Verwendung nach Anspruch 3, wobei der Verlust an Schmierung und/oder struktureller Integrität und/oder die Schwellung auf eine Abreicherung von Phospholipiden zwischen der Sehne oder dem Band und einer Oberfläche, über die sie/es sich bewegt, zurückzuführen ist.

6. Vesikuläre Formulierung zur Verwendung nach einem der Ansprüche 1 bis 5, wobei die verminderte Beweglichkeit durch Viskosupplementation behandelt wird.

7. Vesikuläre Formulierung zur Verwendung nach Anspruch 3 zur Behandlung von Sehnenentzündung (Tendinitis).

8. Vesikuläre Formulierung zur Verwendung nach Anspruch 3 zur Behandlung von Karpaltunnelsyndrom.

9. Vesikuläre Formulierung zur Verwendung nach einem der Ansprüche 1 bis 8, wobei das Tier ein Mensch, ein Begleittier oder ein landwirtschaftliches Tier ist.

10. Vesikuläre Formulierung zur Verwendung nach einem der Ansprüche 1 bis 9, wobei das Tier ein Mensch ist und etwa 45 bis etwa 85 Jahre alt ist.

## Revendications

1. Formulation vésiculaire constituée essentiellement d'un ou de plusieurs phospholipides, d'un ou de plusieurs tensioactifs non ioniques et d'un support pharmaceutiquement acceptable, et éventuellement contenant en outre l'un ou plusieurs parmi les ingrédients suivants : des cosolvants, des agents chélatants, des tampons, des antioxydants, des conservateurs, des microbiocides, des émollients, des humectants, des lubrifiants et des épaississants, pour une utilisation dans le traitement de la mobilité réduite associée à une perte de lubrification et/ou d'intégrité structurale et/ou de gonflement d'une structure de collagène chez un animal en appliquant de manière topique la formulation vésiculaire sur la peau entourant la structure de collagène.

2. Formulation vésiculaire pour une utilisation selon la revendication 1, la structure de collagène étant un cartilage à l'intérieur d'une articulation articulée.

3. Formulation vésiculaire pour une utilisation selon la revendication 1, la structure de collagène étant un tendon ou un ligament.

4. Formulation vésiculaire pour une utilisation selon la revendication 2, la perte de lubrification et/ou d'intégrité structurale et/ou de gonflement étant due à la dégradation de phospholipides sur la surface de cartilage à l'intérieur d'une articulation articulée.

5. Formulation vésiculaire pour une utilisation selon la revendication 3, la perte de lubrification et/ou d'intégrité structurale et/ou de gonflement étant due à l'appauvrissement en phospholipides entre le tendon ou le ligament et une surface sur laquelle il se déplace.

6. Formulation vésiculaire pour une utilisation selon l'une quelconque des revendications 1 à 5, la mobilité réduite étant traitée par viscosupplémentation.

7. Formulation vésiculaire pour une utilisation selon la revendication 3, pour le traitement d'une tendinite.

8. Formulation vésiculaire pour une utilisation selon la revendication 3, pour le traitement du syndrome du canal carpien.

9. Formulation vésiculaire pour une utilisation selon l'une quelconque des revendications 1 à 8, l'animal étant un humain, un animal de compagnie ou un animal agricole.

10. Formulation vésiculaire pour une utilisation selon l'une quelconque des revendications 1 à 9, l'animal étant un humain et étant d'un âge d'environ 45 à environ 85 ans.
